# EUROPEAN PATENT APPLICATION

(11) **EP 4 776 299 A1**
(43) Date of publication of application: **15.07.2026**
(21) Application number: 26150019.3
(22) Date of filing: 02.01.2026
(51) Int. Cl.: G16H 20/40, G16H 40/63, G16H 50/20, G16H 40/20, G16H 20/10

(54) **SYSTEMS AND METHODS FOR REAL-TIME AND RETROSPECTIVE ANESTHESIA PHASE DETECTION**

(30) Priority: 13.01.2025 US 202519019037
(71) Applicant: GE Precision Healthcare LLC, Waukesha, WI 53188 (US)
(72) Inventor: KURUP, Sandeep S., 560066 Bengaluru (IN); ROBIN, Rebinth, 560066 Bengaluru (IN); SUDHA, Sai, 560066 Bengaluru (IN)
(74) Representative: Kilburn & Strode LLP

(57) **Abstract**

Systems are provided for perioperative care. In an example, a system for tracking phases of anesthesia delivery over a case for a patient includes one or more processors and memory storing instructions executable by the one or more processors to output (802) a GUI including a first visual representation of a default or previously-determined phase of anesthesia delivery for the patient; receive (602) a plurality of monitoring parameters of the patient over an observation window; identify (606), by applying a set of rules to the plurality of monitoring parameters, whether an event signaling a change to a new phase of anesthesia delivery for the patient is detected; based on the event being detected and determined to be a true event, update (617) the GUI to display a second visual representation of the new phase; and based on the event not being detected, maintain (810) the first visual representation on the GUI.

## Description

### FIELD

Embodiments of the subject matter disclosed herein relate to patient monitoring during perioperative care, and more specifically to automatic detection of patient anesthesia phase.

### BACKGROUND

Certain medical procedures, such as surgery, may require various sub-procedures to be performed to prep the patient for surgery, maintain the patient in a certain condition during surgery (e.g., anesthetized), and help the patient recover after surgery. Such sub-procedures that are performed in support of a main procedure may be referred to as perioperative care. For a medical facility with multiple operating rooms, for example, monitoring of perioperative care may allow care providers and administrators to effectively manage resources of the medical facility, such as scheduling and preparation of patients for surgery.

### BRIEF DESCRIPTION

In one embodiment, a system for tracking phases of anesthesia delivery over a case for a patient includes one or more processors and memory storing instructions executable by the one or more processors to: output, for display on a display device, a graphical user interface (GUI) including a first visual representation of a default or previously-determined phase of anesthesia delivery for the patient; receive a plurality of monitoring parameters of the patient over an observation window, at least a portion of the plurality of monitoring parameters obtained from an anesthesia delivery machine; identify, by applying a set of rules to the plurality of monitoring parameters, whether an event signaling a change to a new phase of anesthesia delivery for the patient is detected, wherein the event is one of case start, maintenance start, emergence start, and case end; based on the event being detected and determined to be a true event, update the GUI to display a second visual representation of the new phase; and based on the event not being detected, maintain the first visual representation on the GUI.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIGS. 1 and 2 schematically show an example system for perioperative care and supervision configured to automatically detect anesthesia phase.
FIG. 3 shows an example anesthesia delivery machine.
FIG. 4 schematically shows example patient monitoring parameters that may be evaluated to detect an event signaling a change in anesthesia phase.
FIG. 5 schematically depicts a process flow for assigning anesthesia phase based on detected events.
FIG. 6 is a flow chart showing an example method for automatically detecting an event signaling a change in anesthesia phase.
FIG. 7 is a flow chart showing an example method for applying a set of rules to detect an event signaling a change in anesthesia phase.
FIGS. 8A-8C are a flow chart showing an example method for updating a graphical user interface in real-time based on one or more events detected according to the method of FIG. 7.
FIG. 9 is a flow chart showing an example method for updating a graphical user interface retrospectively based on one or more events detected according to the method of FIG. 7.
FIGS. 10-13 show example graphical user interfaces depicting anesthesia phase for a plurality of patients.
FIG. 14 is a flow chart showing an example method for updating a second graphical user interface in real-time based on current anesthesia phase detected according to the method of FIGS. 8A-8C.

### DETAILED DESCRIPTION

Embodiments of systems and methods as disclosed herein operate to facilitate perioperative care for a plurality of patients. To facilitate the perioperative care and supervision described herein, the systems and methods as disclosed herein collect and process medical device data. In particular, the medical device data may be processed in order to identify, for one or more patients being monitored, a current phase of anesthesia delivery. The process of delivering anesthesia may be classified into stages based on key events/timepoints. In particular, the process of anesthesia delivery may be classified based on the start of the process (referred to as case start (CS)), the end of induction (induction end (IE)), which is also the same as the start of maintenance (maintenance start (MS)), the start of emergence (emergence start (SE), which is also the same as the end of maintenance (maintenance end (ME)), and the end of the process (referred to as case end (CE)). Generally, induction refers to the transition from the patient being awake to being in an anesthetized state, maintenance refers to the maintenance of the patient in the anesthetized state, and emergence refers to when anesthesia is no longer needed and the agents administered during the maintenance phase can be switched off.

The current phase of anesthesia delivery may be displayed to one or more users outside the operating room (e.g., a supervising anesthesiologist, an administrator) via a visual dashboard (e.g., graphical user interface). In some examples, the visual dashboard may be displayed via a supervisory application that, in some examples, also facilitates presentation of the medical device data in real-time or near real-time via one or more graphical user interfaces that may be displayed on device(s) of the users, such as a mobile device (e.g., smart phone, tablet, wearable). The supervisory application may facilitate display of medical device data, including physiological data and medical device setting/parameter data, for a plurality of patients and for a plurality of different patient monitoring parameters to the users. The displayed medical device data for the plurality of patients may be displayed simultaneously in a multi-patient graphical user interface (GUI), which may allow the users to easily monitor patient status for each patient, even if the user is located away from the patient(s).

The supervisory application may also monitor patient status, via the medical device data, and may output various notifications, such as alarms, when patient anesthesia phase changes or when a patient has been in a certain phase of anesthesia for longer than a threshold duration.

The visual dashboard and functions of the supervisory application described above may allow for a single supervising care provider and/or administrator to simultaneously monitor multiple patients during respective medical procedures, such as surgery. While each patient may be attended to by multiple care providers during the medical procedure, such as one or more surgeons, nurses, medical technicians, etc., certain supervising care providers, such as anesthesiologists, may attend to multiple patients at once and may oversee a plurality of subordinate care providers, such as nurse anesthesiologists. As the number of subordinate care providers increases relative to the number of supervising care providers, and as medical procedures become more complex, the need for a supervising care provider to be able to monitor patients and oversee subordinate care providers remotely has increased. For example, a supervising anesthesiologist may be scheduled to initiate and monitor an induction phase of anesthesia for a first patient. However, the supervising anesthesiologist may also be attending to six other patients that are in the maintenance and emergence phases of anesthesia. The visual dashboard may notify the supervising anesthesiologist of a relatively long emergence phase for a particular patient, which may indicate delayed recovery due to anesthetic causes (deep anesthesia), which may allow for additional monitoring or action from an anesthesiologist. In doing so, patient care may be improved. In another example, the visual dashboard may indicate insufficient depth of anesthesia during maintenance phase for a patient, which may signal to the anesthesiologist that the patient needs additional monitoring.

Further, the automatic, real-time detection of the current anesthesia phase for each of a plurality of patients may be beneficial for resource utilization at the hospital/medical facility. For example, displaying a summarization of which operating rooms (OR) have patients that are in which anesthesia phases at a given time point may help the hospital administration make correct decisions, such as estimating the wait times for each OR, deciding on staffing requirements, next surgery preparations, etc. As another example, a relatively long time lag between a case start and induction start could indicate a delay in progress of the case due to unavailability of equipment and nurses/anesthesiologists, etc., which may be notified to the hospital administration. Additionally, large time lags between emergence end and case end may indicate logistic issues (patient waiting on bed, lack of manpower). At the same time, in such cases, notifying that the patient is ready for transfer using the patient's anesthesia machine parameters or monitor parameters allows more preparation time for the next patient in queue for entry into the OR.

As used herein, medical device data includes physiological data (also referred to as patient monitoring data) of a patient that is acquired from a medical device and machine data collected internally from the medical device itself. Machine data may include alarms, device status, settings, messages, and measured operational data. Machine data may further include settings and values that represent specific actions taken with the medical device for example, in response to automated controls or due to clinician inputs. For example, in an anesthesia delivery machine, this may include changes to oxygen and/or anesthetic agent concentrations. The machine data may further include clinical and/or technical alarms initiated by the medical device or device diagnostic information. Still further examples of the machine data include proactive or predictive service alerts from the medical device, maintenance checkout information, and/or processor clock cycle signals or power signals or other operational signals from various components of the medical device indicative that the medical device is turned on, in use, in operation, held in a stand by condition, or turned off.

The medical device data can be collected in time series format as provided from the medical devices themselves. As used herein, the time series format of the medical device data can include waveforms, binary data, numeric data, and/or textual data in a time series format. Embodiments of the systems and methods as disclosed herein receive the medical device data from the medical devices at a frequency similar to the frequency at which it is produced by the medical devices. In embodiments, this increased velocity of the received data and the monitoring and analysis of medical device machine data can enable improved monitoring systems and methods as disclosed herein. As described in further detail herein, embodiments of systems and methods support high speed data ingestion, enrichment, normalization, and data curation of the medical device data. The medical device data can undergo real time analysis and further enrichment of the data with event detection and notation.

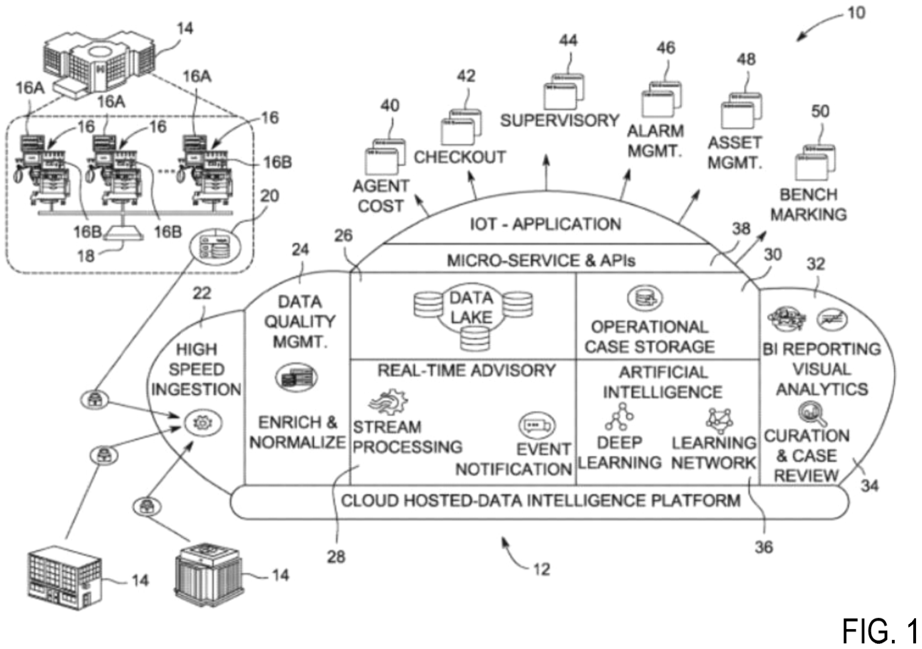

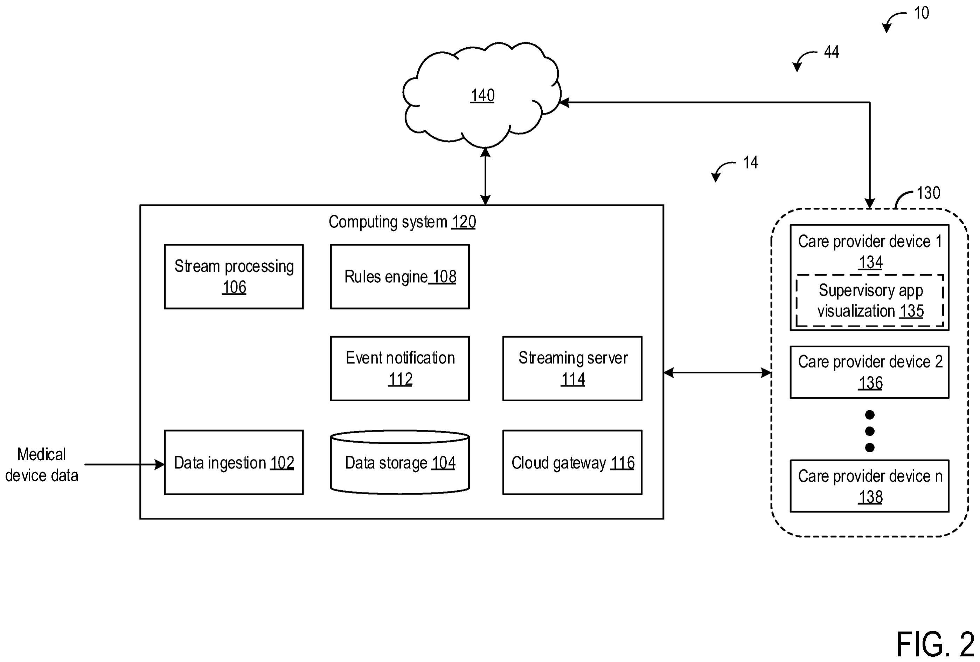

FIGS. 1 and 2 depict an exemplary embodiment of a system 10 for perioperative care and supervision that may be configured to automatically detect and report current anesthesia phase for a plurality of patients. Referring first to FIG. 1, system 10 includes a medical device data (MDD) processing system 12. The MDD processing system 12 can be implemented in a variety of hardware and/or software implementations and it should be noted that such implementations are not considered to be limiting. For example, it is contemplated that any or all of the MDD processing system 12 may be embodied exclusively in hardware, exclusively in software, exclusively in firmware or in any combination of hardware, software, and/or firmware. While the following describes exemplary methods and systems, the examples provided herein are not the only way to implement such methods and systems.

In embodiments that cover an entirely software and/or firmware implementation, in any embodiment, at least one of the elements is hereby expressly defined to include a tangible and non-transient computer readable medium. As used herein, the term tangible computer readable medium is expressly defined to include any type of computer readable storage and to exclude propagating signals. Additionally or alternatively, the example methods and systems may be implemented using coded instruction (e.g., computer readable instructions) stored on a non-transitory computer readable medium such as a flash memory, a read-only memory (ROM) a random-access memory (RAM), a cache, or any other storage media in which information is stored for any duration (e.g. for extended period time periods, permanently, brief instances, for temporarily buffering, and/or for caching of the information). As used herein, the term non-transitory computer readable medium is expressly defined to include any type of computer readable medium and to exclude propagating signals.

In exemplary and non-limiting embodiments of the medical device data processing system 12, the system 12 is implemented by one or more networked processors or computing devices. Processing system 12 may be implemented in a cloud computing platform and/or infrastructure. Memory and processors as referred to herein can be standalone or integrally constructed as part of various programmable devices, including for example, computers or servers. Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include, but are not limited to RAM, ROM, EEPROM, flash memory, CD-ROM, DVD-ROM or other optical storage, magnetic cassettes, magnetic tape, magnetic disc, or other magnetic storage devices, or any other medium which can be used to store the desired electronic format of information and which can be accessed by the processor or processors or at least a portion of a computing device.

The MDD processing system 12 is communicatively connected to at least one hospital network 14. Such communicative connections as well as the hospital network itself may include, but are not limited to, a wide area network (WAN); a local area network (LAN); the internet; wired or wireless (e.g. optical, Bluetooth, radio frequency (RF)) network; a cloud-based computer infrastructure of computers, routers, servers, gateways, etc.; or any combination thereof associated therewith that allows the system or portions thereof to communicate with one or more computing devices.

The hospital network 14 may exemplarily be a network associated with a portion of a hospital, for example a surgery unit or department of a hospital, or may be more broadly located across medical devices of an entire hospital. It further will be recognized that while some embodiments and implementations of the systems and methods as disclosed herein may seek to operate on a single hospital or unit of a hospital, still other embodiments may connect a plurality of hospital networks, including hospitals currently owned or operated or otherwise affiliated with one another. In still further embodiments, while individual hospitals or groups of hospitals may use the MDD processing system 12, the MDD processing system 12 may receive and process information from a plurality of hospital networks including those unaffiliated with one another at the same time.

As depicted in FIG. 1, the hospital network 14 includes a plurality of medical devices 16. The medical devices 16 may include physiological monitoring devices 16a as well as patient therapy devices 16b. Physiological monitoring devices 16a may include, but are not limited to, patient monitors (e.g., heart rate monitors, blood pressure and/or oxygenation monitors, respiration monitors), ECG monitors, EEG monitors, or EMG monitors. An example of an anesthesia delivery machine will be used for discussion purposes as the medical device, and more specifically as the patient therapy device 16b, although it will be recognized by a person of ordinary skill in the art that other devices, including but not limited to patient respiratory assistance devices or dialysis machines, may be further non-limiting examples of patient therapy devices. However, it will be recognized that therapy devices may also include capabilities to not only deliver patient therapy, but also to measure physiological parameters of a patient. For example, embodiments of anesthesia delivery machines may include gas analysis modules operable to measure gas concentrations expired by the patient. In some embodiments, imaging devices, including but not limited to X-ray, CT, MRI, and ultrasound devices, may be examples of medical devices 16 as contemplated within the present disclosure. Still further examples of medical devices may include video and/or audio recording devices.

In some examples, a limited version of the MDD processing system 12 as described herein may be implemented locally, for example as an anesthesia delivery management system 18. In such an embodiment, the anesthesia delivery management system 18 may operate to collect medical device data from a plurality of anesthesia delivery machines 16b *inter alia* to monitor anesthesia agent use between anesthesia delivery machines and across procedures performed by the anesthesia delivery machines in an effort to visualize anesthetic agent consumption and use as well as to quantify, monitor, and evaluate trends across all of the anesthesia delivery machines in the hospital or surgical unit, in addition to the automatic detection of anesthesia phase disclosed herein.

The medical devices 16 may be communicatively connected to one or more edge devices, such as edge device 20. Edge device 20 may exemplarily be an edge processing device, cloud processing device, or internet gateway. The edge device 20 may include an internet of things (IOT) gateway which facilitates a secure communications link between the medical devices 16 at the hospital network 14 with the servers, processors, and computer readable media implementing the MDD processing system 12. In some embodiments, the edge device 20 may communicate directly with one or more of the medical devices 16, or may communicate with the medical devices 16 through an intermediate network, for example, the anesthesia delivery management system 18 or another medical device data system or network.

The edge device 20 receives the medical device data as time series data for any of the medical device data available from the medical devices. As noted above, the data streams of medical device data (e.g., machine data, monitored patient physiological parameter data) are available in time series format as acquired from the medical devices and may include, but are not limited to time series information of alarms, device status, device settings, messages, and measured data. In embodiments, the medical devices may be equipped with sensors that improve the self-awareness of the medical device, e.g. sensors that monitor the function, inputs and/or outputs of various components of the medical device itself. Many such sensors are already incorporated into medical devices such as to measure compressor speeds and/or cycle times, internal pressures, voltages, clock speeds, or temperatures, or other sensors as will be recognized by a person of ordinary skill in the art or as disclosed in further detail herein.

The edge device 20 encrypts the time series formatted data and the encrypted data is transmitted using wired and/or wireless communication techniques for encrypted data to the server, processors, and data storage carrying out the MDD processing system 12. The edge device 20 continuously transmits de-identified medical device data in time series format over an encrypted communication channel to a high speed data ingestion module 22 of the MDD processing system 12. While the embodiment described herein may reference de-identified data, it will be recognized that other embodiments may use patient-identified data with appropriate considerations taken for handling patient data. The high speed data ingestion module 22 takes in the real time streams of medical device data. The data ingestion can be performed in an automated fashion and can preprocess the received streams of real time data in the time series for later processing by the MDD processing system 12. The high speed data ingestion module 22 can receive concurrent data streams from multiple connected devices across multiple sites at a high incoming velocity, for example at or near the frequency at which medical devices can output data. In some embodiments, the high speed data ingestion module 22 is scalable to continue to ingest increased bandwidth of medical device data without significant decrease in ingestion speeds.

The high speed data ingestion module 22 takes the time series medical device data from the medical devices of one or more hospital networks and formats it for further processing by a data quality management module 24. In exemplary and non-limiting embodiments, the high speed data ingestion module 22 supports open standard such as ASTMF 2761 or integrated clinical environmental (ICE). The data quality management module 24 may normalize, enrich, and tag the data streams without negatively impacting data latency. In a healthcare environment, a variety of healthcare information products and/or systems may be used to provide medical services, collect medical data, conduct medical exams, etc. However, many healthcare information systems operate using various messaging standards (e.g., Health Level 7 International (HL7 V2.x/v3), Clinical Document Architecture/Continuity Of Care Document (CDA/CCD), American Society for Testing Materials (ASTM), Digital Imaging and Communications in Medicine (DICOM), etc.)) and various standards and/or protocols (e.g., cross-enterprise document sharing (XDS.A/B) cross-enterprise document media interchange (XDM) cross-enterprise document reliable interchange (XDR), patient identifier cross-referencing/patient demographics query (PIX/PDQ) patient administration management (PAM), query for existing data (QED), national counsel for prescription drug programs (NCPDP), etc.)) that make system integration and/or communication more difficult. Thus, normalization may include reformatting of medical data to a consistent or compatible format for use within the MDD processing system 12. In one embodiment, the medical device data may be normalized into the ISO/IEEE 11073-10101 nomenclature and its extensions. In a still further embodiment, the data quality management module 24 can normalize the streams of incoming time series data by converting units of measure. The data quality management module 24 can further operate to identify and tag various types of medical device data, locations from which the medical device data was received, or time series data streams originating from the same medical device. These tags can be used as further detailed herein to identify and analyze groups of streams of time series data.

In some embodiments, the data quality management module 24 normalizes the received incoming data by transforming and/or translating the clinical data streamed from the source healthcare system or device into a canonical data model with associated metadata. The processed medical device data is stored in a data lake 26 which is exemplarily implemented in computer readable storage embodying capability to store terabytes of data. The data lake 26 is a long-term computer storage repository that holds large amounts of raw data in a native format until the data is needed. The native format may include the time series data from the medical devices which may be in waveform or binary format, audio data, image data, and/or video data. In embodiments, this can help to facilitate the ingestion of the data that may not be processed in real time but may still be taken in in real time or near real time and instead stored in the data lake until further needed. This may be facilitated by identifying particular data streams and limiting the processing of those data streams, for example by the data quality management module 24, if it is known at that time that such data stream is not being used in real time analysis. In an exemplary embodiment, the data quality management module 24 may not convert the data to a canonical data model but may still attempt to tag, enrich, or index the data to facilitate later retrieval of that data in a standardized way from the data lake 26.

In a still further embodiment, portions of the data that are stored in the data lake 26 may also be additionally stored in a graph database which may be a separate database residing on the same computer readable storage, or may be embodied on separate computer readable storage from the data lake 26. The graph database may receive the data streams of which it is known that the system may analyze trends in that data stream. The graph database may store the streams of data in a time series format in a way that facilitates trending of the data over time and appending the data with events either identified in the data itself, in one or more of the other data streams, or received by the system from an external source. These events may include, but are not limited to, medical device or clinician actions, clinical events, situations, or complications that arise during the medical procedure. The graph database may later be used by a clinician or technician to identify further relationships between trends and the data streams with other analysis as disclosed herein.

At the same time that the data is stored in the data lake 26, the enriched and normalized medical device data may be provided to a stream processing engine 28. The stream processing engine 28 identifies cases and events in the time series streams of medical device data. Identified clinical cases may be stored in an operational case database 30. Clinical cases may exemplarily include surgical and intensive care unit (ICU) cases. The clinical cases may be identified by the medical device used and the timing of the medical data in the time series of the medical device data. For example, a time series of medical device data from an anesthesia delivery machine showing a change in status turning the machine on and followed by changes to device settings and delivery and/or consumption of anesthetic agent all indicate that a clinical case has begun or is ongoing.

As noted above, the streams of time series medical device data originating from the same medical device or from the same location in a hospital may be tagged or otherwise identified as being related. These tags can be used to simultaneously analyze related data streams or combine analysis of related data streams to identify clinical cases. For example, a device status data stream analysis may be combined with a user input data stream, device setting data stream, and operational data streams to identify when the device is used and how it is used in the clinical case. This information may help to distinguish between a maintenance or checkout of the medical device by a technician from the use of the device for clinical case.

The analysis of the data streams of multiple medical devices, particularly those identified as being related or co-located may further be used to identify clinical cases. For example, coordinated or similar actions in data streams of an anesthesia delivery device and a related patient monitoring device, and/or respiratory support device and/or imaging device, etc., may further be used to identify that these devices are being used together for a clinical case. In still further embodiments, the streaming time series medical device data may be combined with information regarding scheduled clinical cases to help to further identify when and how the medical devices are used during clinical cases.

In embodiments, knowledge of a scheduled use of the medical device (e.g. anesthesia delivery machine) can be used to further identify clinical cases in the streams of medical device data. For example, input or received knowledge regarding a type and time of a scheduled procedure may help to identify the start and end of the clinical case in particular streams of medical device machine data. In an embodiment, a known schedule of use for the medical device may help to identify clinical cases from maintenance or calibration actions which may similarly require powering up and at least partial operation of the medical device.

The medical device data associated with the actions by the anesthesia delivery machine and/or other medical devices during the identified clinical case may be stored in the operational case database 30. In an example, the identification of the clinical case is stored along with the other time series streams of medical device data from that anesthesia delivery machine as well as time series streams of medical device data from any physiological monitors and/or other medical devices associated with the use of that anesthesia delivery machine. In another exemplary embodiment as described in further detail, a clinical case summary with links or identifiers to the associated time series medical device data stored in the data lake 26 can be created and stored in the operational case database 30.

In an embodiment, prior to storing the clinical cases in the operational case database 30, the clinical cases may be classified or profiled which is a technique used for data curation. The profiling of the clinical cases may be based upon, in part, the information in the clinical case summary. The profiling may be used to group the clinical cases into groups, for example normal cases, edge cases, and outlier cases. These determinations may be made in view of a comparison between the time series data in the clinical case against normal distributions of the same type of time series machine data in other similar clinical cases. Edge cases may be identified as borderline or ambiguous cases, not clearly defined as either normal or an outlier. In an embodiment, for a particular measured value or occurrence, a distribution of such occurrences may be used to establish normal, edge, and outlier cases. In an embodiment, a normal case may be within a standard deviation of a median value in the normal distribution while edge cases are between one and two standard deviations and outlier cases are greater than two standard deviations from the median. The categorized cases, as explained in further detail herein, for example, identified edge cases may be further investigated to create or improve event detection algorithms, rules for clinical decision support, alert algorithms, and predictive algorithms.

The stream processing engine 28 also identifies events in the time series streams of medical device data, for example in the manners as described in further detail herein and presented in reporting and visual analytics tools 32 which exemplarily may be presented on a graphical display communicatively connected to the medical device data processing system 12.

Once clinical cases are stored in the operational case database 30, clinical cases may be reviewed manually by a clinician or technician using a curation and case review tool 34. The curation and case review tool 34 may be presented in a graphical user interface on a graphical display and further provide inputs exemplarily through the graphical user interface for the user or technician to curate or otherwise assess the clinical cases. This can be performed for investigative, educational, and data curation purposes.

The reporting and visual analytics tool 32 can present the detected events in a variety of channels of communication. For example, the detected events may be presented visually through graphical user interfaces and graphical displays. The detected events or notifications of the detected events can also be reported by communication of events/event notifications to wearable or mobile devices and presentation of medical device data and identified events in visual form in reports and/or dashboards presented in a graphical user interface on a graphical display, as will be explained in more detail below.

The results of the streaming analytics and event detection in the time series of medical device data may be provided to an application programming interface (API) 38 for use by application developers to provide monitoring, reporting, and/or control applications based upon the analyzed streams of medical device data. Such applications may operate through a computer operating system, a website browser, or operate on a mobile computing device or wearable computing device. Non-limiting examples of applications that may leverage the analysis of the time series medical device data include, but are not limited to, an anesthetic agent cost dashboard 40, a checkout dashboard 42, a supervisory application 44, an alarm management application 46, an asset management application 48, and a benchmarking application 50.

The agent cost dashboard 40 may present medical device data regarding anesthetic agent use across clinical cases as well as between anesthesia delivery machines within a hospital network or comparatively between hospital networks. By comparatively presenting this information, anesthetic agent use and behavioral changes can be understood and undertaken to promote efficient use of anesthetic agent.

The checkout dashboard 42 may assist in monitoring the inspection and maintenance of the monitored medical devices. Medical device data such as device status and settings, as well as messages and information in machine data, may provide insight into the inspection processes for maintaining medical devices at a hospital network. The checkout dashboard may identify maintenance and/or testing events in the streams of machine data and note these identified testing events against a testing schedule, requirement (e.g., daily), or other criterion.

The supervisory application 44 may be used by attending and/or supervising anesthesiologists to more efficiently manage remote personnel, nurse anesthetists, and/or other care providers simultaneously working across multiple locations or theatres. Additionally or alternatively, the supervisory application 44 may be used by ward or site administrators to more efficiently manage personnel, scheduling, equipment, and the like. The alarm management application 46 may report and present medical device data regarding alarm notifications and silences of alarm notifications in order to better understand and adjust alarms to improve signal to noise in alarm events and to reduce alarm fatigue by clinicians. Additional information about the supervisory application 44 is presented below.

The asset management applications 48 may present use, status, maintenance, and/or inspection information regarding medical devices (e.g. anesthesia delivery machines) or consumables used by medical devices, including components that may be frequently replaced, refilled, or refurbished during normal operation of the medical device (e.g. filters, absorbers). The benchmarking application 50 may provide further operational and quality performance across providers and/or organizations or in a comparative manner, for example, between hospital networks versus averages or between specific locations.

The supervisory application 44 allows for users (e.g., clinicians such as anesthesiologists, nurses, and other care providers as well as administrators) to view anesthesia delivery phase, ventilator, anesthesia, and vital parameters of a plurality of patients in different locations (e.g., in different operating rooms) on various workstations, smart phones, tablets, or other computing devices associated with the users. The supervisory application 44 may include a backend that is hosted on edge device 20 and/or MDD processing system 12 as dockers/micro services and may be rendered on a user's device (such as care provider device 134 shown in FIG. 2) using a suitable visualization platform.

FIG. 2 schematically shows example devices of system 10 via which supervisory application may be executed, including a computing system 120 in communication with a plurality of care provider devices 130 via hospital network 14 and/or via a separate network 140. The computing system 120 may be the edge device 20 and/or the MDD processing system 12 of FIG. 1.

As mentioned above, the computing system 120 receives the medical device data from the medical devices 16. The medical device data received by the computing system 120 may be ingested by a data ingestion module 102, which may be similar to ingestion module 22 of FIG. 1, and stored in data storage 104. Data storage 104 may be an ephemeral datastore where the received data is stored temporarily rather than persistently, or the data storage 104 may include long-term storage. Further, the received medical device data may be allocated to various micro services on the computing system 120 in order to carry out aspects of supervisory application 44, including a stream processing module 106, a rules engine 108, an event notification service 112, a streaming server 114, and a cloud gateway 116.

As explained above, the supervisory application 44 may be used by attending and/or supervising anesthesiologists to manage other care providers, such as nurse anesthesiologists and/or other subordinate care providers. The hospital/medical facility may rely on a relatively high supervision ratio (e.g., 4-10 subordinate care providers for each supervising anesthesiologist), which may increase the need for the supervising anesthesiologists to have high mobility among operating rooms while still overseeing all subordinate care providers and monitoring patient status for all procedures that may be simultaneously ongoing. The supervisory application 44 may facilitate this mobility and management by allowing supervising anesthesiologists to monitor patient status and communicate with subordinate care providers from a remote location. As will be explained in more detail below, the supervisory application 44 may present, via one or more graphical user interfaces displayed on a mobile or other device of a supervising anesthesiologist, patient monitoring parameters (e.g., ECG, heart rate, blood oxygenation) as determined from the received medical device data, procedure phase (e.g., case start, induction, maintenance, emergence, and case end), alarms, anesthesiology machine settings, adequacy of anesthesia, and other relevant or selected information to a user (e.g., the supervising anesthesiologist and/or administrator). The processing and analysis of the time series streams of medical device data as described above in order to detect events relevant to identified cases (e.g., such as identifying a phase of anesthesia administration) may be utilized and the output of such processing and analysis may be provided to the supervisory application 44. The supervisory application 44 may provide determined values of specified patient monitoring parameters, indications of detected events, and other notifications as determined from the time series streams of medical device data to the user via the graphical user interfaces described herein.

For example, via the supervisory application 44, the user may toggle between graphical user interfaces that show limited information for a plurality of patients (a multi-patient GUI), including current procedure phase (e.g., anesthesia delivery phase), and more detailed information for a selected patient (a single patient GUI). The user may also view, via the supervisory application 44, trends of patient monitoring data, detailed alarm/notification information, insights, and/or other information. Further, the user may communicate with other care providers, such as a subordinate care provider that is in the room with a patient, via the supervisory application 44. The user may customize which patients/rooms to view, which patient monitoring parameters to view, which alarms to apply, and other parameters of the graphical user interfaces used to present the above-described information, such as a layout of each graphical user interface.

The graphical user interfaces that are generated via the supervisory application 44 may be displayed on one or more suitable display devices associated with a respective care provider device and/or medical facility administration device. As shown in FIG. 2, a plurality of care provider devices 130 may be included as part of hospital network 14, from a first care provider device 134, a second care provider device 136, and on up to an nth care provider device 138, and may be communicatively coupled to computing system 120 via hospital network 14. Each care provider device may include a processor, memory, communication module, user input device, display (e.g., screen or monitor), and/or other subsystems and may be in the form of a desktop computing device, a laptop computing device, a tablet, a smart phone, or other device. Each care provider device may be adapted to send and receive encrypted data and display medical information, including medical images in a suitable format such as digital imaging and communications in medicine (DICOM) or other standards. The care provider devices may be located locally at the medical facility and substantially fixed in place (such as in a nurses' station or in the room of a patient) and/or located locally or remotely from the medical facility and configured to move with the care provider (such as a care provider's mobile device).

When viewing graphical user interfaces generated via the supervisory application 44 via a display of a care provider device, a care provider may enter input (e.g., via the user input device, which may include a keyboard, mouse, microphone, touch screen, stylus, or other device) that may be processed by the care provider device and sent to computing system 120. In examples where the user input is a selection of a link or user interface control button of a graphical user interface, the user input may trigger progression to a desired view or state of the graphical user interface (e.g., trigger display of desired patient medical information), trigger updates to the configuration of the graphical user interface, trigger alarm, insight, and/or other notification settings to be saved, trigger changes to a machine (such as an anesthesia delivery machine), or other actions.

The devices disclosed herein, such as the care provider devices and/or aspects of the computing system 120, may each include a communication module, memory, and processor(s) to store and execute aspects of the supervisory application 44 as well as send and receive communications, graphical user interfaces, medical data, and other information.

Each communication module facilitates transmission of electronic data within and/or among one or more systems. Communication via the communication module can be implemented using one or more protocols. In some examples, communication via the communication module occurs according to one or more standards (e.g., Digital Imaging and Communications in Medicine (DICOM), Health Level Seven (HL7), ANSI X12N, etc.). The communication module can be a wired interface (e.g., a data bus, a Universal Serial Bus (USB) connection, etc.) and/or a wireless interface (e.g., radio frequency, infrared, near field communication (NFC), etc.). For example, a communication module may communicate via wired local area network (LAN), wireless LAN, wide area network (WAN), etc. using any past, present, or future communication protocol (e.g., BLUETOOTH^{®}, USB 2.0, USB 3.0, etc.).

Each memory may include one or more data storage structures, such as optical memory devices, magnetic memory devices, or solid-state memory devices, for storing programs and routines executed by the processor(s) to carry out various functionalities disclosed herein. Memory may include any desired type of volatile and/or non-volatile memory such as, for example, static random access memory (SRAM), dynamic random access memory (DRAM), flash memory, read-only memory (ROM), etc. The processor(s) may be any suitable processor, processing unit, or microprocessor, for example. The processor(s) may be a multi-processor system, and, thus, may include one or more additional processors that are identical or similar to each other and that are communicatively coupled via an interconnection bus.

As used herein, the terms "sensor," "system," "unit," or "module" may include a hardware and/or software system that operates to perform one or more functions. For example, a sensor, module, unit, or system may include a computer processor, controller, or other logic-based device that performs operations based on instructions stored on a tangible and non-transitory computer readable storage medium, such as a computer memory. Alternatively, a sensor, module, unit, or system may include a hard-wired device that performs operations based on hard-wired logic of the device. Various modules or units shown in the attached figures may represent the hardware that operates based on software or hardwired instructions, the software that directs hardware to perform the operations, or a combination thereof.

"Systems," "units," "sensors," or "modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

One or more of the devices described herein may be implemented over a cloud or other computer network. For example, computing system 120 is shown in FIG. 2 as constituting a single entity, but it is to be understood that computing system 120 may be distributed across multiple devices, such as across multiple servers.

The supervisory application 44 may provide various data, notifications, and messages to the plurality of care provider devices 130. The data, notifications, and/or messages may include historical data, real-time medical device data (e.g., provided by streaming server 114), and notifications that may pushed to the plurality of care provider devices 130 from an event notification service 112.

As will be explained in more detail below, the supervisory application 44 may be visualized on a care provider device in the form of one or more graphical user interfaces. The one or more graphical user interfaces may be populated with real-time patient monitoring parameters, including a current phase of anesthesia delivery. In some examples, the one or more graphical user interfaces may further be populated with other real-time patient monitoring parameters, such as most-recently determined values or waveforms for heart rate, blood oxygen saturation, respiration rate, and so forth, obtained from the medical devices. When the medical device data is received by the computing system 120, some or all of the medical device data may be processed by stream processing module 106 and supplied to the streaming server 114, which may then supply the real-time patient monitoring parameter values and/or waveforms to a requesting care provider device. For example, when a user is viewing a patient-specific graphical user interface of the supervisory application 44 on care provider device 134, the graphical user interface may include tiles or other display areas where the most-recently determined values for selected patient monitoring parameters are displayed. The streaming server 114 may stream the most-recently determined values for the selected patient monitoring parameters to the care provider device 134, which may then populate the received values into the graphical user interface. The stream processing module 106 may include rule-based streaming analytics algorithms applying windowing functions (sliding, tumbling, hopping, etc.) used for waveform analysis and event detection, thereby triggering alerts, detection of surgical phases, flow analysis, triaging algorithms, etc.

The determination of which patient monitoring parameter values to send to which care provider device may be based at least in part on data requests sent by the care provider devices to the computing system 120. The computing system 120 may include a representational state transfer (REST) server, for example, that may receive data requests from the care provider devices 130 and may respond to the data requests by commanding the streaming server 114 to stream selected medical device data to a requesting care provider device(s). The streaming server 114 may maintain a stateful session (e.g., WebSocket) with each client (e.g., the care provider devices). The medical device data may be adapted (transformed and filtered) before being streamed to the client devices.

The supervisory application 44 may generate and/or send various alarms and notifications based on the medical device data received from the various medical devices. The alarms may include threshold-based alarms, where a notification/alarm is generated and output to one or more care provider devices in response to a patient monitoring parameter value meeting a predetermined condition relative to a threshold (e.g., an alarm may be generated and sent to a care provider device in response to a duration of an emergence phase for a particular patient exceeding a threshold duration). Computing system 120, via event notification service 112 and/or cloud gateway 116, may send a notification of the alarm to the care provider device of the care provider attending to the patient and/or to a care provider device of an administrator. For example, the alarms that are generated may be sent to the appropriate care provider device(s) directly via event notification service 112 or via the cloud gateway 116, which may push the alarms (and other notifications that are generated by computing system 120, as explained in more detail below) to the appropriate care provider device(s), even when the supervisory application 44 is in an unlaunched state on the care provider device(s).

The rules engine 108 may include resources (e.g., memory and processors) of the computing system 120 allocated to store and apply sets of anesthesia event detection rules, which may be applied to the medical device data in order to automatically determine whether an event signaling a change in anesthesia phase has occurred, for each of a plurality of patients, in real-time or near real-time. As mentioned above, the current phase of anesthesia delivery may be identified based on a set of events (CS, MS, ES, and CE). To correctly detect the above events, sliding windows of time-series medical device data (e.g., anesthesia delivery machine data) are collected as input and rule-based logic is deployed to detect each event in real-time. A previous state/event need not be maintained to support a state-less architecture (with low input/output cost). Each input parameter can come at a variable frequency which can further be interpolated in the module. Patient case can be parsed with a given window size (preferably small, e.g., 1 minute) with a slide (e.g., 1 second). In other words, for each machine and/or monitoring parameter, a time-aligned window of data (e.g., a minute of data) may be collected and the sets of event detection rules may be applied on each window of data to identify if an event is detected, which may be repeated with new windows of data at a given frequency (e.g., each second). Additional details about the event detection rules and machine and monitor parameters that may be evaluated to determine the current phase are provided below.

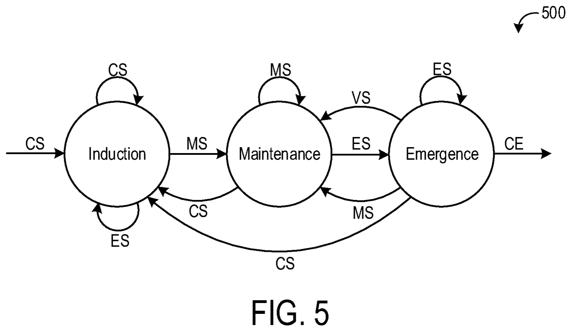

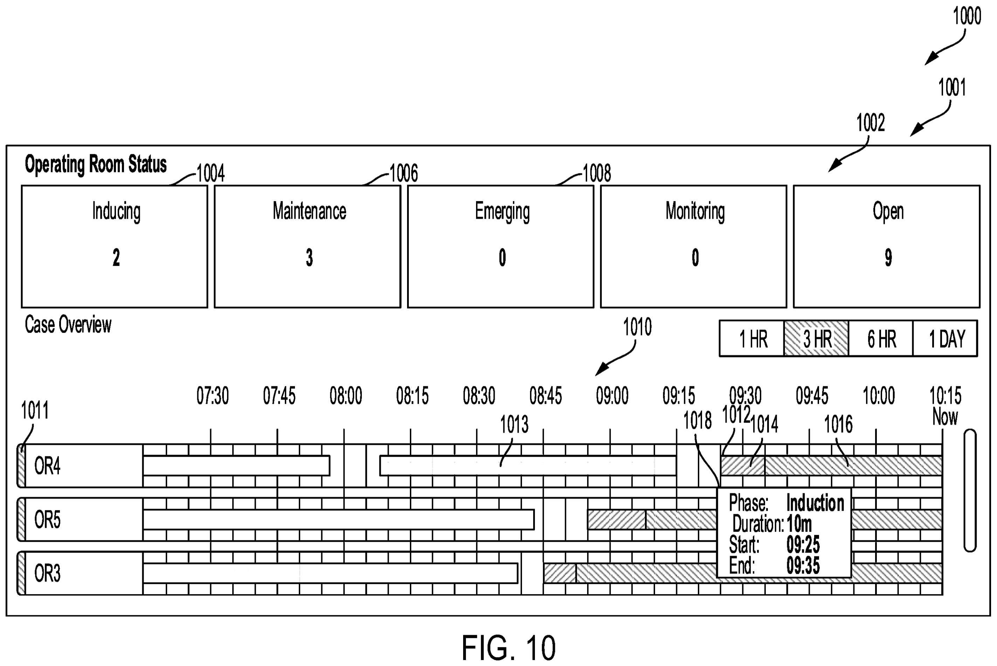

The computing system 120 may distribute medical device data streams to the rules engine 108, and the rules engine 108 may apply the stored event detection rules to the incoming streams of medical device data in order to determine if any phase notifications or results (e.g., indicating a change to a new phase of anesthesia delivery) should be generated. If a phase notification is to be generated, a phase notification may be generated and used to update a GUI to indicate the new phase of anesthesia delivery, with the GUI viewable via the appropriate care provider device(s). Example methods for applying the event detection rules to detect an event and update a GUI are presented below with respect to FIGS. 5-9, and example GUIs that may be displayed to illustrate anesthesia phase for a plurality of patients is shown in FIGS. 10-13.

The computing system 120 can be implemented in a variety of hardware and/or software implementations and it should be noted that such implementations are not considered to be limiting. For example, it is contemplated that any or all of the computing system 120 may be embodied exclusively in hardware, exclusively in software, exclusively in firmware, or in any combination of hardware, software, and/or firmware. The examples provided herein are not the only way to implement such methods and systems.

In exemplary and non-limiting embodiments of the computing system 120, the computing system 120 is implemented by one or more processors or computing devices. Memory and processors as referred to herein can be standalone or integrally constructed as part of various programmable devices, including for example, computers or servers. Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include, but are not limited to, RAM, ROM, EEPROM, flash memory, CD-ROM, DVD-ROM or other optical storage, magnetic cassettes, magnetic tape, magnetic disc, or other magnetic storage devices, or any other medium which can be used to store the desired electronic format of information and which can be accessed by the processor or processors or at least a portion of a computing device.

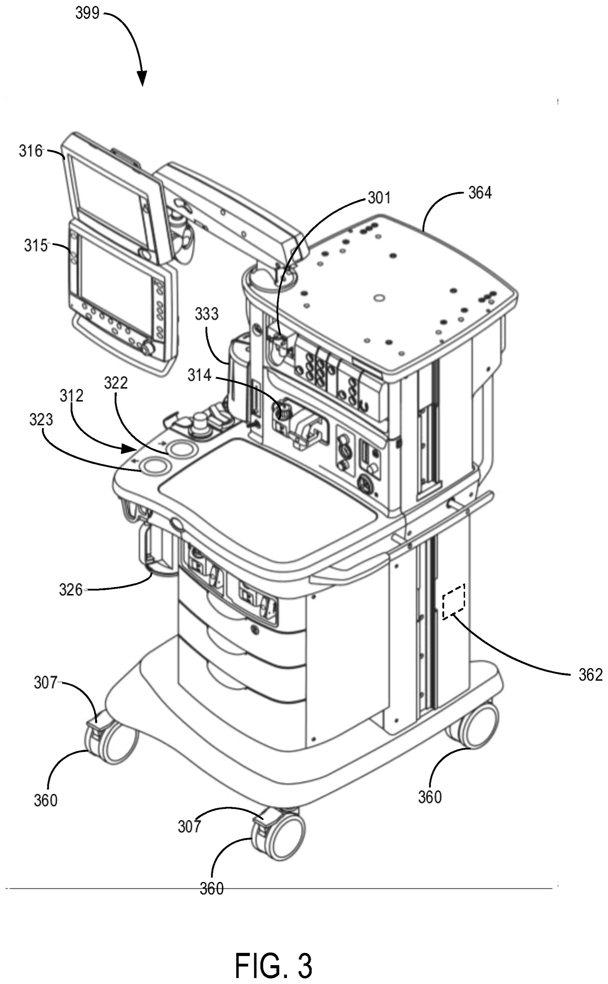

FIG. 3 shows an example an anesthesia machine 399, which is a non-limiting example of a patient therapy device 16b of FIG. 1. Anesthesia machine 399 includes a frame 364 supported by casters 360, where the movement of the casters may be controlled (e.g., stopped) by one or more locks 307. In some examples, the frame 364 may be formed of a plastic material (e.g., polypropylene). In other examples, the frame3 64 may be formed from a different type of material (e.g., metal, such as steel).

Anesthesia machine 399 also includes respiratory gas module 301, ventilator 312 (explained in more detail below), vaporizer 314 (explained in more detail below), anesthesia display device 315, and patient monitoring display device 316.

Vaporizer 314 may be in the form of one or more removable cassettes such that a supply of anesthetic agent can be removed from the cassette and such that different types of anesthetic agents can be supplied to the anesthesia machine by simply removing the cassette and replacing it with a different cassette for a different anesthetic agent. When more than one anesthetic agent is to be delivered, more than one cassette may be included with a different anesthetic agent in each cassette. The vaporizer 314 includes a housing having a drug reservoir that contains a supply of anesthetic agent to be delivered to a patient. The drug reservoir may have a discharge opening formed in a back wall of the housing that is configured to receive a discharge tube (not shown), which is part of the anesthesia machine, forming a gas-tight seal for delivery of anesthetic vapor to a patient.

Vaporizer 314 may include at least one absorbent wick within the reservoir. A passageway allows fresh gas from a gas source to pass to the absorbent wick(s). Vaporized liquid from the absorbent wick(s) and accompanying fresh gas may flow to the back of the drug reservoir and out the discharge opening in the housing. A lower portion of the drug reservoir may contain the liquid anesthetic agent and an upper portion of the reservoir may contain the vaporized anesthetic agent and breathing gases. During operation, a combination of temperature and pressure affect the liquid anesthetic agent and cause it to vaporize into the breathing gases. The gases carrying the vaporized agent are then discharged through the discharge opening.

A rear of the anesthesia machine 399 may include one or more pipeline connections to facilitate coupling of the anesthesia machine to pipeline gas sources. Additionally, the rear of the anesthesia machine may include a cylinder yoke, via which one or more gas-holding cylinders may be coupled to the anesthesia machine. Thus, through the pipeline connection and/or cylinder connections, gas may be provided to the anesthesia machine, where the gas may include but is not limited to fresh gas, oxygen, and nitrous oxide. The gas that enters the anesthesia machine may mix with the vaporized anesthetic agent at the vaporizer, as described above, and be supplied to a patient via the ventilator. In some embodiments, the rear of the anesthesia machine may also include a serial port, a collection bottle connection, cylinder wrench storage, anesthesia gas scavenging system, main power inlet, system circuit breaker, equipotential stud, outlet circuit breaker, and isolated electrical outlet.

The ventilator 312 may include an expiratory check valve 322, inspiratory check valve 323, absorber canister 326, and bellows assembly 333. When a patient breathing circuit is coupled to the ventilator, the breathing gases (e.g., fresh gas, oxygen, and/or nitrous oxide mixed with vaporized anesthetic agent) exit the machine from an inspiratory port (which may be positioned at the same location as the inspiratory check valve 323) and travel to a patient. Expiratory gases from the patient re-enter the anesthesia machine via an expiratory port (which may be positioned at the same location as the expiratory check valve 322), where the carbon dioxide may be removed from the expiratory gases via the absorber canister 326.

During operation of the vaporizer 314, an operator (e.g., an anesthesiologist) may adjust an amount of vaporized anesthetic agent that is supplied to a patient by adjusting a flow rate of gases from the gas source(s) (e.g., the gas pipelines) to the vaporizer. The flow rate of the gases from the gas source to the vaporizer may be adjusted by the operator via adjustment of one or more flow adjustment devices. For example, the flow adjustment devices may include analog and/or digital adjustment dials and/or other user input devices configured to actuate one or more flow control valves of the anesthesia machine 399. In one example, a first flow control valve may be positioned between the gas source(s) and the vaporizer 314 and may be actuatable via the flow adjustment devices to a fully opened position, a fully closed position, and a plurality of positions between the fully opened position and the fully closed position.

The anesthesia machine may additionally include one or more bypass valves configured to bypass gases from the gas source(s) around the vaporizer 314. The bypass valves may enable a first portion of gases flowing from the gas source to flow directly from the gas source to the inspiratory port, and a second portion of gases flowing from the gas source may flow through the vaporizer 314 to mix with the vaporized anesthetic agents prior to flowing to the inspiratory port. By adjusting a ratio of an amount of gases flowing to the port via the bypass valves relative to an amount of gases flowing to the port via the vaporizer 314, the operator may control a concentration of vaporized anesthetic agent in gases at the port.

Further, the adjustments described above may be facilitated at least in part based on output from the respiratory gas module 301. Respiratory gas module 301 may be configured to measure various parameters of the gases exiting the vaporizer and/or being provided to the patient. For example, respiratory gas module 301 may measure the concentrations of carbon dioxide, nitrous oxide, and anesthesia provided to the patient. Further, respiratory gas module 301 may measure respiration rate, minimum alveolar concentration, patient oxygen, and/or other parameters. The output from the respiratory gas module 301 may be displayed via a graphical user interface displayed on a display device (e.g., device 315 and/or 316) and/or used by the controller to provide closed-loop feedback control of the amount of anesthesia provided to the patient.

Ventilator 312 may optionally be coupled to a breathing circuit (not shown) including a plurality of tubes (e.g., gas passages). The breathing circuit may be coupled between an airway of a patient (e.g., via a breathing mask positioned to enclose the mouth and/or nose of the patient) and the inspiratory port. Gases (e.g., oxygen, or a mixture of oxygen and vaporized anesthetic agents from vaporizer 314) may flow from the port, through the breathing circuit, and into the airway of the patient, where the gases are absorbed by the lungs of the patient. By adjusting the concentration of vaporized anesthetic agent in the gases as described above, the operator may adjust an amount by which the patient is anesthetized.

During conditions in which the breathing circuit is coupled to the airway, anesthetic agent and/or fresh gases may flow into the airway (e.g., be inhaled by the patient) via the inspiratory check valve 323. For example, inspiratory check valve 323 may open automatically (e.g., without input or adjustment by the operator) in response to inhalation of the patient, and may close automatically in response to exhalation of the patient. Similarly, the expiratory check valve 322 may open automatically in response to exhalation of the patient, and may close automatically in response to inhalation of the patient.

In some examples, the operator may alternately and/or additionally control one or more operating parameters of the anesthesia machine 399 via an electronic controller 362 of the anesthesia machine 399 (shown schematically by FIG. 3). Controller 362 includes a processor operatively connected to a memory. The memory may be a non-transitory computer-readable medium and may be configured to store computer executable code (e.g., instructions) to be processed by the processor in order to execute one or more routines. The memory may also be configured to store data received by the processor. Controller 362 may be communicatively (e.g., wired or wirelessly) coupled to one or more external or remote computing devices, such as computing system 120, and may be configured to send and receive various information, such as measured patient monitoring parameters (e.g., the output from the respiratory gas module 301), electronic medical record information, procedure information, and so forth.

The controller receives signals from the various sensors of the anesthesia machine 399 and employs the various actuators of the anesthesia machine 399 to adjust operation of the anesthesia machine 399 based on the received signals and instructions stored on the memory of the controller. For example, the flow of gases to the inspiratory port may be controlled via an input device (e.g., keyboard, touchscreen, etc.) coupled to the electronic controller of the anesthesia machine 399. The controller may be electrically coupled to display device 315 and/or 316 in order to display operating parameters of the anesthesia machine 399. The controller may receive signals (e.g., electrical signals) via the input device and may adjust operating parameters of the anesthesia machine 399 in response (e.g., responsive) to the received signals. For example, the operator may input a desired flow rate of gases (e.g., oxygen) flowing from the gas source to the patient and/or vaporizer 314.

A corresponding valve position of one or more valves of the anesthesia machine (e.g., a position of one or more bypass valves, as described above) may be empirically determined and stored in a predetermined lookup table or function on the controller. For example, the controller may receive the desired flow rate of gases via the input device and may determine an amount of opening of the one or more bypass valves corresponding to the desired flow rate based on the lookup table, with an input being the desired flow rate and an output being the valve position. The controller may transmit an electrical signal to an actuator of the valve in order to adjust the valve position. In some examples, the controller may compare the desired flow rate of gases to a measured flow rate of gases as measured by a flow rate sensor (e.g., an inspiratory flow sensor).

Controller 362 is shown in FIG. 3 for illustrative purposes, and it is to be understood that controller 362 may be located internally of anesthesia machine 399 and thus may not be visible externally on anesthesia machine. Controller 362 may include multiple devices/modules that may be distributed across anesthesia machine 399.

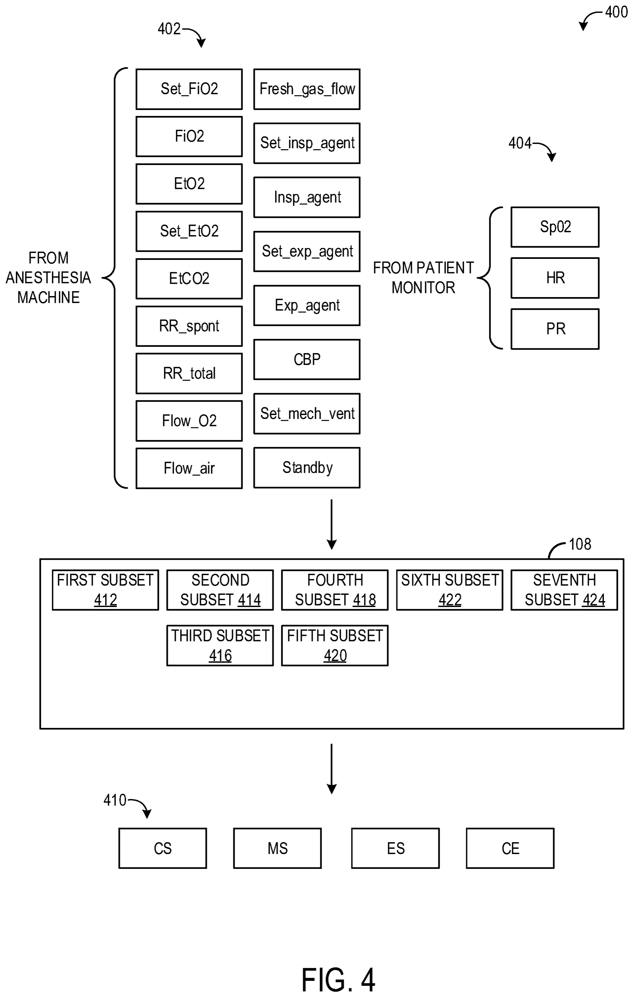

FIG. 4 schematically shows example monitoring parameters 400 (e.g., medical device data) that may be processed via the computing system 120 using phase detection rules of rules engine 108 in order to determine whether an event signaling a change to a new phase of anesthesia delivery has occurred. The parameters 400 may be non-limiting examples of the medical device data discussed above with respect to FIGS. 1-3. The parameters 400 may include a first set of parameters 402 that are obtained from an anesthesia delivery machine (e.g., anesthesia machine 399), which may include one or more of measured fraction of inspired oxygen (FiO2); measured end-tidal oxygen (EtO2); measured end-tidal CO2 (EtCO2); set FiO2 (Set_FiO2); set EtO2 (Set_EtO2); spontaneous respiration rate (RR_spont); total respiration rate (RR_total); set mechanical ventilation (Set_mech_vent); set inspired anesthetic agent (Set_insp_agent); set expired anesthetic agent (Set_exp_agent); measured inspired anesthetic agent (Insp_agent); measured expired anesthetic agent (Exp_agent); fresh gas flow rate (Fresh_gas_flow); O2 flow rate (Flow_O2); air flow rate (Flow_air); cardiac bypass mode (CBP); and anesthesia machine standby state (standby). It should be appreciated that different anesthesia delivery machines may output different monitoring parameters. For example, some anesthesia delivery machines may not output the set FiO2 (Set_FiO2), the set EtO2 (Set_EtO2), and/or the set inspired anesthetic agent (Set_insp_agent) or the set expired anesthetic agent (Set_exp_agent). As a specific example, a premium or new-generation anesthesia delivery machine having a first configuration may output all the parameters listed above, while a first prior-generation anesthesia delivery machine having a second configuration may output all the parameters listed above other than the set inspired anesthetic agent (Set_insp_agent) and the set expired anesthetic agent (Set_exp_agent). A second prior-generation anesthesia delivery machine having a third configuration may output all the parameters listed above other than the set FiO2 (Set_FiO2), the set EtO2 (Set_EtO2), the set inspired anesthetic agent (Set_insp_agent), and the set expired anesthetic agent (Set_exp_agent).

The parameters 400 may optionally include a second set of parameters 404 that are obtained from one or more patient monitoring devices not included as part of the anesthesia machine, including one or more of oxygen saturation measurement (SpO2), heart rate (HR), and pulse rate (PR).

The rules engine 108 may apply a set of rules to determine if an event of a plurality of events 410 is detected, wherein the event signals a change to a new phase of anesthesia delivery. The plurality of events 410 may include case start (CS), which may signal the start of induction, start of maintenance (maintenance start, MS), start of emergence (emergence start, ES), and case end (CE). Specifically, each event may have one or more subsets of rules that can be applied to detect that event.

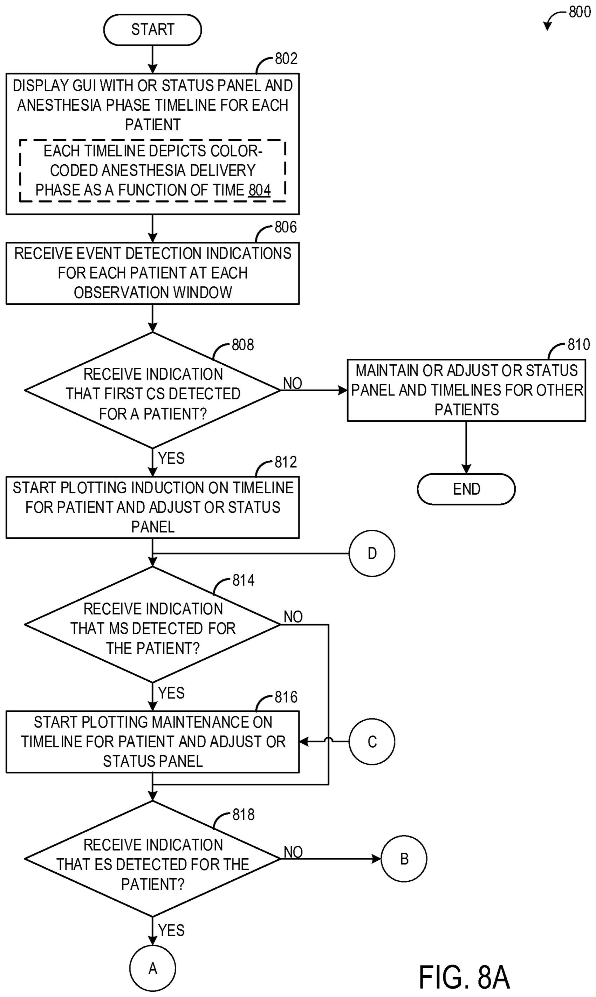

As shown, the rules engine 108 includes seven subsets of rules. A first subset 412 of rules may be applied to detect case start and govern the updating of one or more GUIs in real-time based on detection of case start. The first subset 412 of rules, when applied to the monitoring parameters 400 over an observation window, may indicate CS responsive to the anesthesia machine standby state ending (e.g., the anesthesia machine standby switched from being in standby to standby being turned off/ending). The first subset 412 of rules may further govern the updating of one or more GUIs, such that when the CS event is detected for a patient, the patient may be indicated to be in induction, and induction may be plotted on a timeline for that patient, among other changes to GUIs to reflect the patient is in induction. Additional details about updating one or more GUIs based on detecting that a patient is in induction are provided below with respect to FIGS. 8A-8C and 10-13. Additionally, the first subset 412 of rules may dictate that any CS event detected between an initial CS and a CE event results in the previous CS event being discarded and a new case being started.

A second subset 414 and a third subset 416 of rules may be applied to detect maintenance start and govern the updating of one or more GUIs in real-time based on detection of maintenance start. The second subset 414 of rules, when applied to the monitoring parameters 400 over an observation window, may indicate MS responsive to a mechanical ventilation start event (e.g., set mechanical ventilation going from off/zero to a valid value) being present in the observation window and at least one valid value of EtCO2 (e.g., EtCO2 greater than 0.2) being present during the observation window. The third subset 416 of rules, when applied to the monitoring parameters 400 over an observation window, may indicate MS responsive to detection of a particular total flow pattern in the observation window. The total flow pattern may include two consecutive values of total flow in the observation window being equal to or greater than a total flow threshold (e.g., 5 L/min), at least three of the next five total flow values being less than the total flow threshold, and the third total flow value (e.g., the value immediately after the first two total flow values) being less than the total flow threshold. Thus, the total flow pattern may include, over seven total flow values received during the observation window, the first two values (of the seven) being equal to or greater than the total flow threshold, the third value being less than the total flow threshold, and at least two of the fourth through seventh values being less than the total flow threshold. In some examples, the total flow values may be fresh gas flow values.

The second subset 414 may be applied in parallel with the third subset 416 to ensure robust detection of maintenance start. Once MS is detected via the second subset 414 or the third subset 416, the patient may be indicated to be in maintenance, and maintenance may be plotted on a timeline for that patient, among other changes to GUIs to reflect the patient is in maintenance. Additional details about updating one or more GUIs based on detecting that a patient is in maintenance are provided below with respect to FIGS. 8A-8C and 10-13.

A fourth subset 418 and a fifth subset 420 of rules may each be applied to detect emergence start and govern the updating of one or more GUIs in real-time based on detection of emergence start. The fourth subset 418 of rules, when applied to the monitoring parameters 400 over an observation window, may indicate ES responsive to a mechanical ventilation end event (e.g., set mechanical ventilation going from on to off) being present in the observation window (and lasting for at least a specified duration, such as 55s) and the anesthesia machine not being run in cardiac bypass mode. The fifth subset 420 of rules, when applied to the monitoring parameters 400 over an observation window, may indicate ES responsive to two of the following three conditions being true/satisfied over the observation window: increase in O2 percentage (e.g., FiO2 or set_FiO2 increasing), increase in total flow (e.g., fresh gas flow) by more than a threshold (e.g., 4L/min), and an end of anesthetic agent delivery (e.g., which may be detected based on set or measured inspired anesthetic agent).

The fourth subset 418 may be applied in parallel with the fifth subset 420 to ensure robust detection of emergence. Once ES is detected via the fourth subset 418 or the fifth subset 420, the patient may be indicated to be in emergence, and emergence may be plotted on a timeline for that patient, among other changes to GUIs to reflect the patient is in emergence. Additional details about updating one or more GUIs based on detecting that a patient is in maintenance are provided below with respect to FIGS. 8A-8C and 10-13. Additionally, the fourth subset 418 and the fifth subset 420 of rules may dictate that emergence be indicated only if the current phase is maintenance and that any MS event (including any mechanical ventilation start event) detected during emergence results in reversion to the previous phase (e.g., maintenance).

A sixth subset 422 of rules may each be applied to detect case end and govern the updating of one or more GUIs in real-time based on detection of case end. The sixth subset 422 of rules, when applied to the monitoring parameters 400 over an observation window, may indicate CE responsive to the anesthesia machine standby state starting (e.g., the anesthesia machine switched from being in active mode to standby mode). The sixth subset 422 of rules may further govern the updating of one or more GUIs, such that when the CE event is detected for a patient, the case for the patient may be indicated as being completed, and the timeline for that patient may be done, among other changes to GUIs to reflect the case has ended. Additional details about updating one or more GUIs based on detecting CE for a patient are provided below with respect to FIGS. 8A-8C and 10-13.

Thus, the first through sixth subsets of rules may be applied to each observation window for each patient/OR/anesthesia machine to track, in real-time, the current anesthesia phase for each patient. One or more GUIs may be updated in real-time (e.g., as each case is ongoing and responsive to each event detection without intentional delay) based on the event detection.

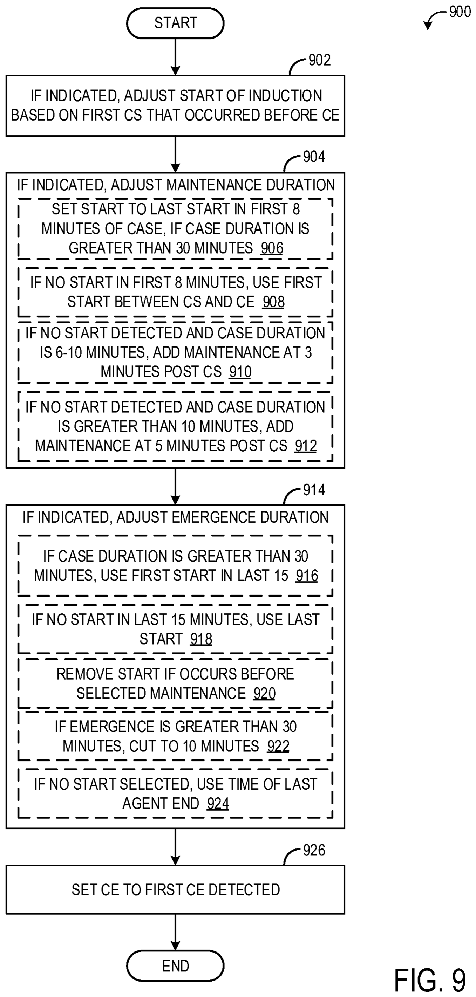

A seventh subset 424 of rules may be applied retrospectively (e.g., after an active case is complete) to govern the updating of a summary view of the case on one or more GUIs based on detection of each event and timing of each detection. The seventh subset 424 of rules, which may also be referred to as summary rules, may dictate which CS, MS, ES, and/or CE event to use (in the event multiple CS, MS, ES, and/or CE events are detected for a given case/patient) to mark the start, end, and transition between phases for each completed case. Further, the seventh subset 424 of rules may dictate when MS and/or ES should be added to the summary in cases where MS and/or ES were not detected at all, as well as other fringe/edge case scenarios. Additional details about updating a summary view of one or more GUIs based on the summary rules for a patient are provided below with respect to FIG. 9.

The rules stored within the rules engine 108 and applicable to detect the events described herein may also account for certain use cases that may otherwise trigger false event detections or missed event detections. For example, some clinicians may turn on and off the mechanical ventilator at the very beginning of a case to confirm the ventilator works properly. The rules applied to detect maintenance may include the mechanical ventilator being started while the rules applied to detect emergence may include the mechanical ventilator being turned off. Thus, the mechanical ventilator being turned on and then off at the beginning of a case may send mixed signals that may result in the induction and maintenance being truncated and skipping almost immediately to emergence. The rules stored within the rules engine 108 may account for this, and avoid false event detection, by restarting the case in maintenance when a second mechanical ventilation start is detected and/or by not allowing the case to transition to emergence owing to the short maintenance phase.

Thus, the method for phase detection disclosed herein uses anesthesia machine parameters to detect events signaling a change in anesthesia phases as well as CS and CE events. In contrast, existing solutions only assess brain activity dependent states or depend on explicit clinician inputs. Accordingly, the method disclosed herein does not require additional patient devices (e.g., EEG devices), and thus may be less expensive, more comfortable for patients, and/or more accurate and faster than existing solutions, as well as allowing for detection of all events including case start and case end. Further, the method for phase detection disclosed herein relies on a relatively small number of anesthesia machine parameters that are available on any anesthesia machine configuration and without requiring parameters from a separate patient monitor. For example, detection of each phase may be accomplished based only on seven parameters (e.g., FiO2, EtCO2, fresh gas flow, measured inspired anesthetic agent, CBP mode, set mechanical ventilation, and standby setting), which may simplify processing relative to other methods for detecting anesthesia phase and reduce the amount of data that needs to be transferred to the rules engine 108. Moreover, the proposed method is a real-time and state-less multi-parametric approach which would work for all types of anesthesia machines, special cases such as cardiac bypass, total intravenous cases (TIVA cases), and with or without monitor data availability. By being state-less, the proposed method does not rely on a previously-determined anesthesia phase but instead identifies whether or not an event is detected at each observation window (e.g., sliding window of received parameters), without knowing the current or previous anesthesia phase or previously-identified event. In doing so, the proposed method may demand less memory and may not be unduly influenced by prior false event detections, thereby increasing processing efficiency and system performance. It is to be appreciated that the event determination based on the current observation window may be stateless, but the handling of the event determination for the purposes of updating one or more GUIs may depend on knowing the current and/or previous anesthesia phase (e.g., in the case where emergence is plotted on the timeline only if the current phase is maintenance and ES has been detected).

FIG. 5 schematically depicts a process flow 500 for transitioning between anesthesia phases for a case/patient based on events detected by applying a set of rules to anesthesia machine parameters, as explained above with respect to FIG. 4. A CS event is detected by applying the first subset 412 of rules, as explained above, which indicates the case is currently in induction. During induction (e.g., before an MS event is detected), there may be situations where a CS event is detected or an ES event is detected, such as when a clinician operating the anesthesia machine turns the mechanical ventilation on, then off, then back on. In such cases, induction may be restarted or maintained, as shown by the arrows pointing back to induction in response to a CS or ES event being detected.

When in induction and an MS event is detected by applying the second subset 414 and the third subset 416 of rules, the case may be indicated as currently being in maintenance. During maintenance, there may be situations where an MS event is again detected or a CS event is detected. If an MS event is detected while in maintenance, maintenance is maintained (as shown by the arrow pointing back to maintenance in response to an MS event being detected). If a CS event is detected while in maintenance, the current case is deemed complete and a new case is started (e.g., in induction), as shown by the arrow pointing back to induction in response to a CS event being detected during maintenance.

When in maintenance and an ES event is detected by applying the fourth subset 418 and the fifth subset 420 of rules, the case may be indicated as currently being in emergence. During emergence, there may be situations where an ES event is again detected, or a CS event, MS event, or vent start (VS) event is detected. If an ES event is detected while in emergence, the case is maintained in emergence (as shown by the arrow pointing back to emergence in response to an ES event being detected). If an MS or VS event is detected while in emergence, the case reverts back to maintenance (as shown by the arrows pointing back to maintenance in response to an MS or VS event being detected). If a CS event is detected while in emergence, the current case is deemed complete and a new case is started (e.g., in induction), as shown by the arrow pointing back to induction in response to a CS event being detected during emergence.

Once a CE event is detected by applying the sixth subset 422 of rules, the case is deemed complete, at any point that the CE event is detected (e.g., regardless of the current phase). The process flow 500 depicted in FIG. 5 may dictate how the one or more GUIs are updated based on detected events, as explained in more detail below.

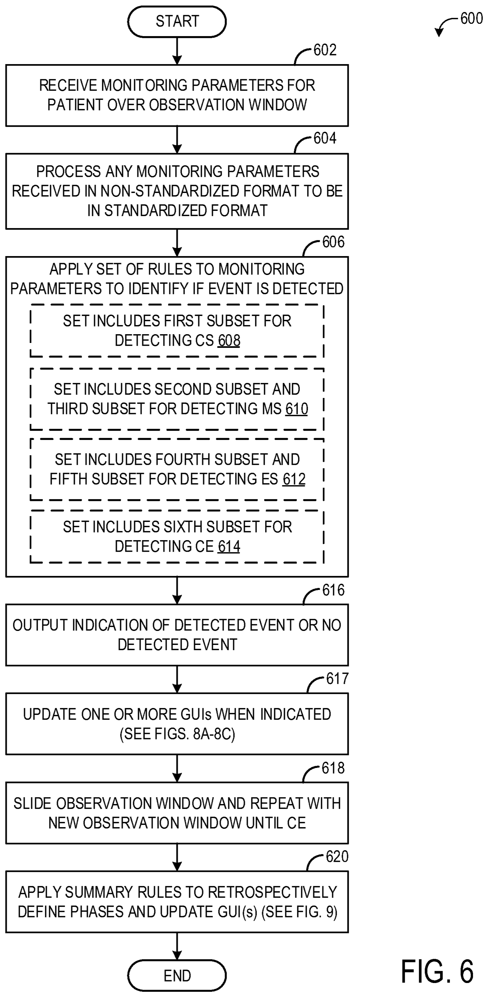

FIG. 6 shows a high-level method 600 for detecting anesthesia delivery events for a patient. Method 600 may be implemented with the system 10 of FIGS. 1 and 2. Method 600 may be carried out according to instructions stored in non-transitory memory and executed by one or more processors, such as memory and processor(s) of computing system 120 of FIG. 2.

At 602, method 600 receives monitoring parameters for a patient over an observation window. The monitoring parameters may be received from an anesthesia delivery machine, such as anesthesia machine 399 of FIG. 3. The monitoring parameters may include some or only a subset of the monitoring parameters 400 described above with respect to FIG. 4. Further, as explained above with respect to FIGS. 1 and 2, the monitoring parameters may be ingested by the computing system and stored (at least temporarily). The observation window may include a set period of time over which the monitoring parameters are received and stored for further processing to detect an event, as will be explained below. The observation window may thus include a suitable duration of time, such as one minute. The observation window may be selected to allow sufficient time to detect changes in selected monitoring parameters that are indicative of an event signaling a new phase of anesthesia delivery, yet short enough to minimize the overall amount of data stored and allow for rapid event detection (e.g., avoid undue delays in detecting a shift to a new phase).

At 604, may method 600 includes processing any monitoring parameters received in a non-standardized format to be converted into a standardized format. The processing into the standardized format may be performed in order to prepare the received monitoring parameter data for evaluation via a set of rules of the rules engine to detect an event, as explained in more detail below, and thus the standardized format may include a format usable by the rules engine. The processing may include interpolating one or more monitoring parameters to include a standardized number of data points. For example, some monitoring parameters may be recorded and/or sent at a relatively low frequency (e.g., once every 10 seconds) compared to other monitoring parameters that may be recorded and/or sent at a relatively high frequency, such as once per second. Thus, the monitoring parameters recorded and/or sent at the lower frequency(ies) may be interpolated to increase the number of data points for those monitoring parameters over the observation window. In this way, any missing values in the raw data from the anesthesia machine and optionally patient monitor may be replaced by forward filling the raw data in the observation window with a prior segment of data (e.g., the prior 30 seconds' worth of data) to maintain data validity and assist parameter comparison. Other examples of processing may include averaging, filtering, determination of trends (e.g., change in a monitoring parameter over the observation window), etc. Still other processing may be applied, such as conversion to a standardized communication protocol. For example, some anesthesia machines may communicate according to a first protocol while other anesthesia machines may communicate according to a second protocol, and the processing may include converting monitoring parameters received in accordance with the second protocol to the first protocol.

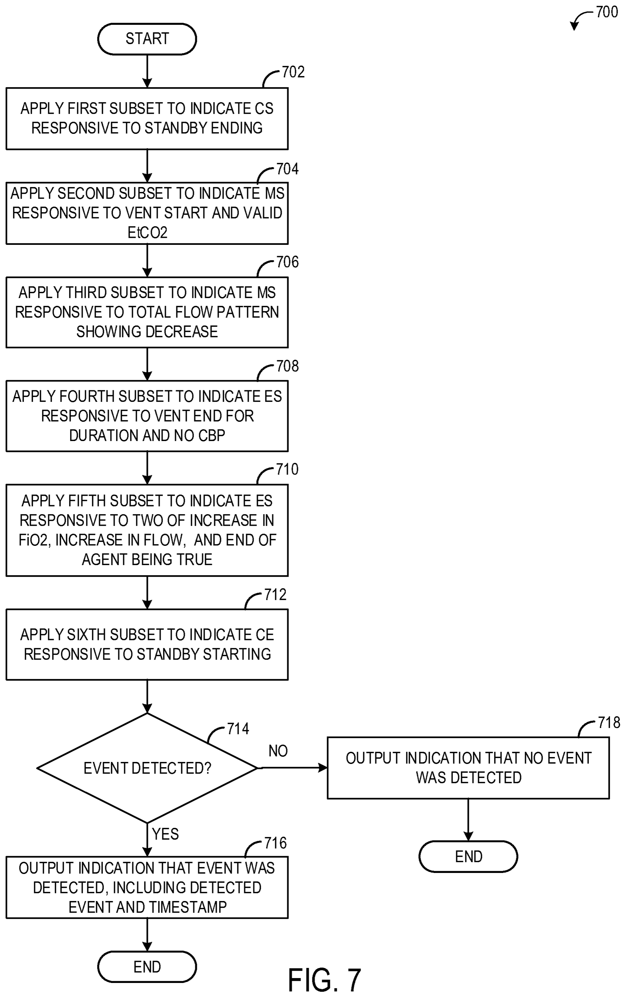

At 606, method 600 includes applying a set of rules to detect if an event has occurred, based on the received monitoring parameters. As indicated at 608, the set of rules may include a first subset of rules for detecting case start (e.g., the first subset 412). As indicated at 610, the set of rules may include a second subset of rules and a third subset of rules for detecting maintenance start (e.g., the second subset 414 and the third subset 416). The selected set of rules may include a fourth subset and a fifth subset of rules for detecting emergence start (e.g., the fourth subset 418 and the fifth subset 420), as indicated at 612. As indicated at 614, the set of rules may include a sixth subset of rules for detecting case end (e.g., the sixth subset 422). Additional details about applying the set of rules to the monitoring parameters to identify if an event is detected are provided below with respect to FIG. 7. As explained previously, the events that may be detected include events that signal a change to a new phase of anesthesia delivery, and as such may be somewhat transient events. Accordingly, for each observation window, an output may be generated that indicates that an event was detected (and if so, which event) or that no event was detected, as indicated at 616, depending on the result of the set of rules applied to the monitoring parameters. When no event is detected, the new phase of anesthesia delivery has not happened yet and the current phase of anesthesia delivery may be ongoing. However, the method for identifying if an event has occurred as described herein with respect to FIG. 6 may be stateless, in that a prior or current anesthesia phase is not known. Accordingly, when an event is not detected, the indication that is output may not include the current anesthesia phase.

At 617, method 600 includes updating one or more graphical user interfaces (GUIs) when indicated, based on the output indication. Additional details about updating the one or more GUIs are provided below with respect to FIGS. 8A-8C. Briefly, each time an event is detected, a GUI (such as the GUI shown in FIG. 10) may be updated to reflect transition to a new phase of anesthesia delivery. The GUI(s) may be updated in real-time, e.g., as anesthesia delivery/the case is ongoing.

At 618, method 600 includes sliding the observation window and repeating the method (e.g., receiving the monitoring parameters for the patient over the new observation window, processing the monitoring parameters, applying the set of rules, outputting the indication, and updating the one or more GUIs), which may be repeated until case end is detected. The observation window may be slid by a suitable amount, such as one second. In this way, the indication of whether or not an event was detected may be repeated over the course of the anesthesia delivery for the patient with newly-obtained monitoring parameters at a desired frequency (e.g., each second), using the past one minute (or other suitable duration) of received monitoring parameters. At 620, method 600 includes applying summary rules (e.g., the seventh subset 424) to retrospectively define the anesthesia phases. Additional details about applying the summary rules are provided below with respect to FIG. 9. It is to be appreciated that method 600 may be performed for each patient/operating room undergoing, about to undergo, or ending anesthesia delivery at a given medical facility, unit, or ward.

Turning now to FIG. 7, a method 700 is shown for applying a set of rules to received monitoring parameters of a patient in order to detect an event signaling a change in anesthesia delivery to the patient. Method 700 may be implemented with the system 10 of FIGS. 1 and 2. Method 700 may be carried out according to instructions stored in non-transitory memory and executed by one or more processors, such as memory and processor(s) of computing system 120 of FIG. 2. In some examples, method 700 may be carried out as part of method 600, e.g., at 606 of method 600.

At 702, method 700 includes applying the first subset of rules to indicate CS responsive to the standby setting of the anesthesia machine ending. The first subset of rules may be applied over the observation window such that if the standby setting is switched off at any point during the observation window (e.g., thereby indicating the anesthesia machine is active), CS is detected.

At 704, method 700 includes applying the second subset of rules to indicate MS responsive to ventilation start and a valid EtCO2 measurement. The second subset of rules may be applied over the observation window such that if mechanical ventilation is started at any point during the observation window, followed by a valid EtCO2 measurement (e.g., where EtCO2 is above a threshold, such as 0.2 mmHg), MS is detected.

At 706, method 700 includes applying the third subset of rules to indicate MS responsive to a total flow pattern showing a decrease in total flow over the observation window. The total flow pattern may include observation of seven consecutive fresh gas flow values, with the first two fresh gas flow values of the seven consecutive fresh gas flow values being equal to or above a flow threshold (5L/min), followed by a third fresh gas flow value that is below the flow threshold, and at least two of the next three fresh gas flow values being below the flow threshold. The third subset of rules may be applied over the observation window such that if the total flow pattern is detected at any point during the observation window, MS is detected. Further, the second subset and the third subset may be applied in parallel, and MS may be indicated in response to whichever subset first indicates MS.

At 708, method 700 includes applying the fourth subset of rules to indicate ES responsive to ventilation ending for a duration and no CBP mode. The fourth subset of rules may be applied over the observation window such that if mechanical ventilation is ended at any point during the observation window and stays off for a duration of the observation window (e.g., 55 seconds), while the anesthesia machine is not being operated in the cardiac bypass mode, ES is detected.

At 710, method 700 includes applying the fifth subset of rules to indicate ES responsive to two of the following parameters being true: an increase in FiO2 (e.g., oxygen percentage), an increase in total flow (e.g., an increase in fresh gas flow), and an end of anesthetic agent delivery. The fifth subset of rules may be applied over the observation window such that if two of the three parameters are observed at any point during the observation window, ES is detected. Further, the fourth subset and the fifth subset may be applied in parallel, and ES may be indicated in response to whichever subset first indicates ES.

At 712, method 700 includes applying the sixth subset of rules to indicate CE responsive to the standby setting of the anesthesia machine starting. The sixth subset of rules may be applied over the observation window such that if the standby setting is switched on at any point during the observation window (e.g., thereby indicating the anesthesia machine is in standby mode), CE is detected.

At 714, method 700 determines if an event is detected, wherein the event is one of case start, maintenance, emergence start, and case end, by applying the set of rules to the received monitoring parameters as explained above. If an event is detected (e.g., case start is detected as explained above, maintenance start is detected as explained above, emergence start is detected as explained above, or case end is detected as explained above), method 700 proceeds to 716 to output an indication that an event was detected. The indication may include an identification of the event that was detected and a time stamp indicating the time at which the event was detected. The indication may be saved in memory (e.g., of computing system 120) and/or sent to one or more computing devices (e.g., first care provider device 134). The indication may be used to update one or more GUIs, as will be explained below with respect to FIGS. 8A-8C.

If an event is not detected (e.g., none of case start, maintenance start, emergence start, or case end is detected), method 700 proceeds to 718 to output an indication that no event was detected. The indication may be saved in memory (e.g., of computing system 120) and/or sent to one or more computing devices (e.g., first care provider device 134). When no event is detected, the indication may be used to maintain a current state of the one or more GUIs with respect to the patient, as will be explained below.

In some examples, two or more subsets of rules of the set of rules may be applied simultaneously to the received monitoring parameters, which may expedite the event detection. In such examples, an entirety of method 700 may be performed for each observation window of each patient, regardless of whether an event is detected or not. For example, method 700 may be performed to apply the set of rules to monitoring parameters received over an observation window for a patient and case start may be detected. Despite case start being detected, the remaining aspects of method 700 may still be carried out to determine if any other events are detected. In other examples, the subsets of rules of the set of rules may be applied serially (e.g., one after the other). In such examples, the next subset of rules may only be applied if an event was not detected in the current subset of rules. For example, the method may be terminated once an event is detected (e.g., once case start is detected, any remaining subsets of rules not yet applied may not be applied), which may also expedite event detection by eliminating unnecessary continued event detection.

FIGS. 8A-8C show a method 800 for displaying a current anesthesia phase for each of a plurality of patients. Method 800 may be implemented with the system 10 of FIGS. 1 and 2. Method 800 may be carried out according to instructions stored in non-transitory memory and executed by one or more processors, such as memory and processor(s) of computing system 120 of FIG. 2. In some examples, method 800 may instead be carried out according to instructions stored in non-transitory memory and executed by one or more processors of a computing system in communication with computing system 120 of FIG. 2, such as a care provider device (e.g., first care provider device 134). In some examples, method 800 may be carried out as part of method 600, e.g., at 617 of method 600.

At 802, a graphical user interface (GUI) is displayed (e.g., on a display device included as part of communicatively coupled to the computing system) that includes an operating room (OR) status panel and anesthesia phase timeline for each of a plurality of patients. The plurality of patients may be undergoing, or about to undergo, anesthesia delivery as part of a surgical or other medical procedure at a specific medical facility being tracked by the computing system. For example, each patient of the plurality of patients may be located in a respective operating room of the medical facility. The OR status panel may include a summary of current anesthesia delivery phase for the plurality of patients, such as the number of patients in induction, the number of patients in maintenance, and the number of patients in emergence. The OR status panel may further include a number of operating rooms that are open (e.g., do not currently have a patient accommodated therein). Each anesthesia phase timeline depicts color-coded anesthesia phases as a function of time, as indicated at 804. For example, a first anesthesia phase timeline for a first patient may begin at case start, which may signal the start of induction and may be indicated on the timeline in a first color, and transition to maintenance 10 minutes after case start, wherein maintenance may be indicated on the timeline in a second color. Example GUIs including OR status panels and timelines for a plurality of patients are shown in FIGS. 10-12.

At 808, method 800 includes receiving an event detection indication for each patient at each observation window. The indications may be generated according to method 600 and method 700, for example. As explained above, an event may include one of case start, maintenance start, emergence start, and case end. The method to identify if an event has occurred (e.g., method 600 and method 700) may be executed for each patient that is about to start receiving anesthesia or is currently receiving anesthesia. In some examples, rather than identifying the patients themselves, the method may be performed for each operating room or each anesthesia delivery machine that is in operation as a proxy for identifying a patient. As explained above with respect to FIG. 6, during each observation window, for each patient (or operating room or anesthesia delivery machine), an indication may be generated of whether an event has been detected for that patient or whether an event has not been detected for that patient. The computing system executing method 800 may receive each indication for each observation window for each patient, and each indication may be time-stamped. In some examples, each indication may be stored in a table or other data structure. Alternatively, once an indication indicating case start has been detected for a given patient is received, the table may be updated to indicate the current phase of anesthesia delivery (e.g., maintenance) and time the current phase started. The current phase may be maintained until an indication is received that a new phase for the given patient has been detected, at which point the table may be updated to indicate that the current phase of anesthesia delivery (e.g., emergence) and the time at which the new phase started for the given patient. In either example, the table may store the current phase of anesthesia delivery for each patient as well as each known prior phase and the timing/duration of each phase.

At 808, method 800 includes determining if an indication that a first CS has been detected for a selected patient has been received. If no, method 800 proceeds to 810 to maintain or adjust the OR status panel and timelines of the GUI for the other patients, based on detected events. For example, the method may include determining whether any of the indications (e.g., received at 806) indicate that an event (or other than the first CS for the selected patient) was detected, or determine whether all the indications indicate no event was detected. If an indication that an event for a patient has been detected is not received (e.g., the most-recently received indications all indicate that no event was detected), the OR status panel and each timeline may be maintained in the current state, other than advancing time for each timeline. For example, referring back to the first timeline for the first patient discussed above, the first timeline may continue to indicate that the first patient is undergoing induction, as no event indicating otherwise has been detected. If an event for a patient is detected, the OR status panel may be updated and the timeline for that patient adjusted to reflect the new phase. This process may continue until each case is determined to be complete. Method 800 then ends. It is to be appreciated that the process described below (e.g., updating the GUI for the selected patient based on the first CS event detected for that patient and then subsequently updating the GUI as further events are detected for the selected patient) is performed for each patient.

If an indication that a first CS has been detected for a selected patient is received, method 800 proceeds to 812 to start plotting induction on the timeline for the selected patient and adjust the OR status panel of the GUI (e.g., to increase the number of patients currently in induction). At 814, method 800 includes determining if an indication that MS has been detected for the selected patient is received. Throughout the course of execution of method 800, indications may be received for each patient, including the selected patient. When one of the indications includes MS being detected for the selected patient, method 800 proceeds to 816 to start plotting maintenance on the timeline for the selected patient and adjust the OR status panel of the GUI (e.g., to increase the number of patients currently in maintenance and reduce the number of patients currently in induction). If an indication that MS has been detected for the selected patient is not received, method 800 proceeds to 818.

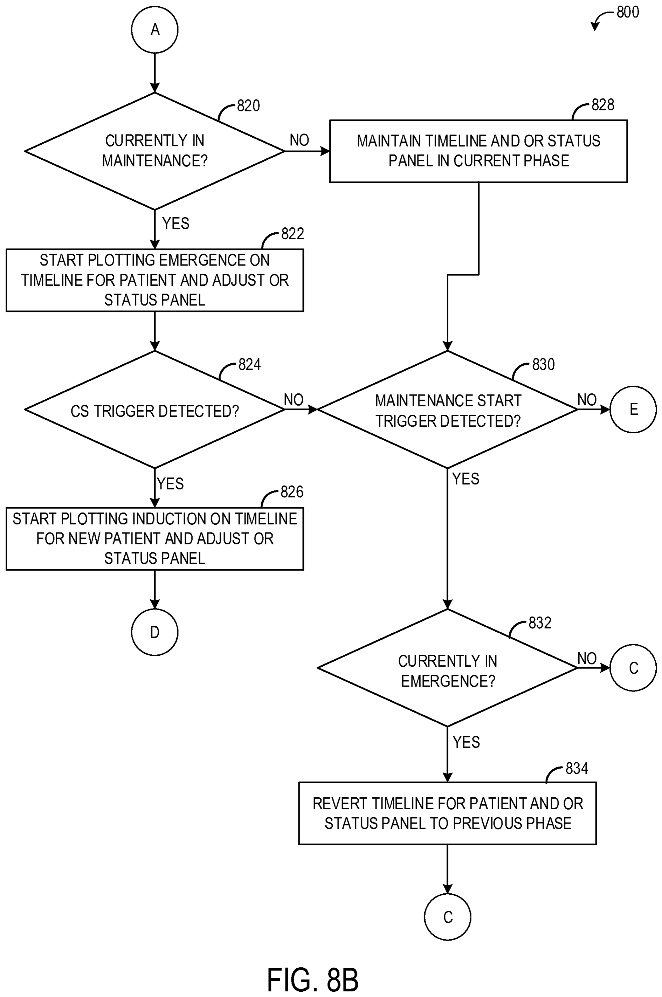

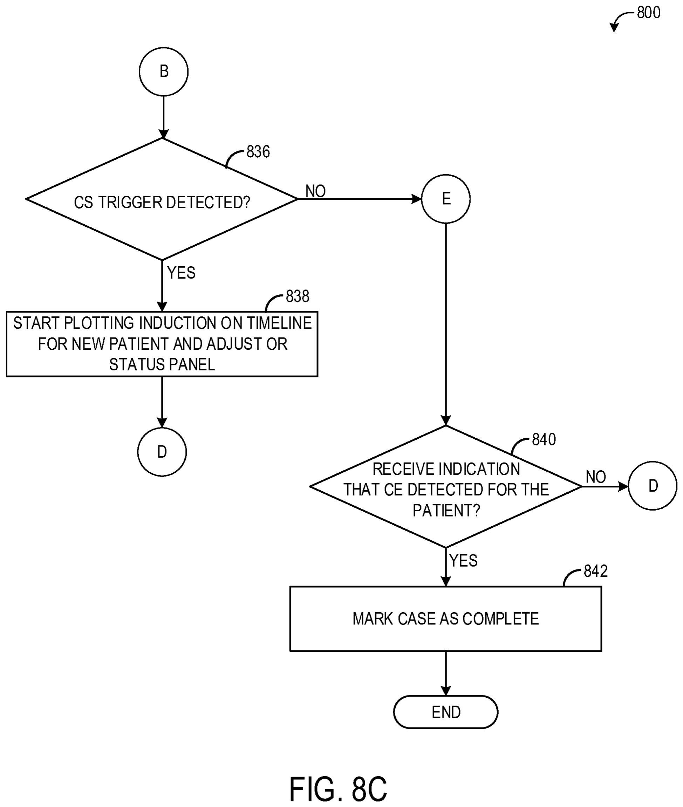

At 818, method 800 includes determining if an indication that ES has been detected for the selected patient is received. If an indication that ES has been detected for the selected patient is not received, method 800 proceeds to 836 of FIG. 8C, which is explained in more detail below. If an indication that ES has been detected for the selected patient is received, method 800 proceeds to 820 of FIG. 8B. At 820, method 800 includes determining if the selected patient is currently in maintenance. As explained above, some actions may trigger detection of ES even if the patient is not actually transitioning from maintenance to emergence. Thus, the determination of whether the patient is currently in maintenance when the ES is detected for the patient may help mitigate false ES event detections.

If the selected patient is not currently in maintenance, the ES event detection may not be a true detection and thus method 800 may proceed to 828 to maintain the timeline for the selected patient in the current phase and maintain the OR status panel. If the selected patient is currently in maintenance, the ES event detection may be a true detection and thus method 800 may proceed to 822 to start plotting emergence on the timeline for the selected patient and update the OR status panel of the GUI (e.g., to increase the number of patients currently in emergence and reduce the number of patients currently in maintenance).

At 824, method 800 determines if a CS trigger is detected. As explained above, a CS trigger may include the anesthesia machine standby mode being switched off, and may be determined based on an indication being received that a CS event for the selected patient has been detected. If a CS trigger is detected, method 800 proceeds to 826 to start plotting induction on a timeline for a new patient/case and adjust the OR status panel. At any point while tracking the anesthesia phases of the selected patient, if a CS event is detected, the current case for the selected patient may be deemed complete and a new case may be started (e.g., for a new patient). If a CS trigger is not detected, method 800 proceeds to 830 to determine if a maintenance start trigger has been detected (e.g., if an indication has been received that a MS event is detected for the selected patient). As explained above, MS may be detected based on ventilation starting and/or based on the particular flow pattern being observed. It is possible that maintenance start can be detected while the selected patient is determined to be in emergence. Thus, if a maintenance start trigger is not detected, method 800 proceeds to 840 of FIG. 8C, which is explained in more detail below, and if a maintenance start trigger is detected, method 800 proceeds to 832 to determine if the selected patient is currently in emergence. If the selected patient is not currently in emergence, method 800 proceeds to 816 to start plotting maintenance on the timeline for the patient and adjust the OR status panel, as explained above. When a maintenance start event is detected and the selected patient is not emergence, then the selected patient is determined to be in maintenance and the GUI is updated accordingly.

If at 832 it is determined that the patient is currently in emergence, method 800 proceeds to 834 to revert the timeline for the selected patient back to the previous phase (e.g., maintenance) and adjust the OR status panel accordingly (e.g., reduce the number of patients in emergence). As such, when a MS event is detected while the selected patient is currently in emergence, the emergence event that triggered the transition to emergence may be considered a false event and the selected patient may be reverted back to maintenance on the GUI.

Returning to 818 of FIG. 8A, if it is determined, after receiving the indication that CS has been detected for the selected patient, and optionally also receiving the indication that MS has been detected for the selected patient, that an indication that ES has been detected for the selected is not received, method 800 proceeds to 836 (shown in FIG. 8C), where method 800 includes determining if a CS trigger is detected, which is similar to the determination of 824 explained above. When the selected patient is currently in induction or maintenance and a CS trigger is detected (e.g., the anesthesia machine is removed from standby mode), method 800 proceeds to 838 to start plotting induction on a timeline for a new patient/case and adjust the OR status panel. At any point while tracking the anesthesia phases of the selected patient, if a CS event is detected, the current case for the selected patient may be deemed complete and a new case may be started (e.g., for a new patient). If a CS trigger is not detected, method 800 proceeds to 840 to determine if an indication that a CE event has been detected has been received. If a CE event is not detected for the selected patient, method 800 proceeds to 814 to continue monitoring for an MS event. Basically, once the initial CS event for the selected is detected and induction begins to be plotted for the selected patient on the GUI, method 800 iterates to determine if any events are detected for the selected patient. During a standard case with no false event detections or atypical events (e.g., patient suddenly removed from the anesthesia machine), method 800 will proceed, after detecting the initial CS event, to eventually detect an MS event (and start plotting maintenance on the timeline of the GUI for the selected patient). During the interval between when the initial CS event is detected and the MS event is detected, the selected patient is assumed to be in induction and the timeline is maintained to reflect that. Once the selected patient is in maintenance, method 800 will proceed to eventually detect an ES event (and start plotting emergence on the timeline of the GUI for the selected patient). During the interval between when the MS event is detected and the ES event is detected, the selected patient is assumed to be in maintenance and the timeline is maintained to reflect that. However, method 800 includes checks and actions for when events are detected that deviate from the order presented above.

Returning to 840, if a CE event is detected for the selected patient, method 800 proceeds to 842 to mark the case as complete. When the case is marked as complete, the timeline for the selected patient is finished and the OR status panel is adjusted to reflect that the selected patient is no longer in the current phase (e.g., emergence). During a standard case as explained above, the CE event is detected after the selected patient has been in emergence for a duration. However, it is possible that a CE event can be detected while the selected patient is in a different phase (e.g., in a non-standard case), and thus 842 may be performed at any point during the case when a CE event is detected. Method 800 then ends.

FIG. 9 shows a method 900 for retrospectively updating one or more anesthesia phases for a patient by applying summary rules (e.g., the seventh subset 424 of rules). Method 900 may be implemented with the system 10 of FIGS. 1 and 2. Method 900 may be carried out according to instructions stored in non-transitory memory and executed by one or more processors, such as memory and processor(s) of computing system 120 of FIG. 2. In some examples, method 900 may instead be carried out according to instructions stored in non-transitory memory and executed by one or more processors of a computing system in communication with computing system 120 of FIG. 2, such as a care provider device (e.g., first care provider device 134). In some examples, method 900 may be carried out as part of method 600, e.g., at 620 of method 600. Method 900 is described below with respect to a patient whose case has been marked complete based on execution of method 800 above. In some examples, method 900 may be performed responsive to an indication that the case has been marked complete for the patient. Method 900 may be executed for all patients/cases once their cases have been marked as complete in order to update the timeline on the GUI for that patient. It is to be appreciated that in addition to updating the timeline that is displayed on the GUI, the timeline may be saved in long-term memory for later viewing and insight gathering. Further, the events and relative timing of the events may be used for various downstream processing tasks, such as adequacy of anesthesia or other algorithms.

At 902, method 900 includes, if indicated, adjusting the start of induction for the patient based on the first CS event that occurred/was detected before a CE event was detected for that patient. As explained above with respect to FIGS. 8A-8C, while the case is actively ongoing for the patient, induction may be plotted on the timeline for the patient upon detection of the first CS event. However, some cases may include more than one CS event. If more than one CS event was detected for the patient, the timeline for the patient (which is still displayed on the GUI, at least for a duration, after the case has ended, as will be explained below) may be adjusted so that induction starts upon the first CS event detected before the CE event for that patient. For the real-time updating of FIGS. 8A-8C, whenever a CS event is detected for a case, the prior events are discarded and plotting starts anew, and thus multiple CS events are not shown on the real-time timeline. However, for the retrospective analysis of FIG. 9, all CS events are recorded, and when more than one CS event is detected, the last CS event that occurred before CE is selected so that the live/real-time and retrospective data are in sync.

At 904, method 900 includes, if indicated, adjusting the duration of maintenance. The duration of the maintenance phase may be adjusted when multiple MS events are detected or when no MS event is detected for the case. Adjusting the duration of maintenance may include setting the start of maintenance to the last MS detected in the first 8 minutes of the case, if the case duration is greater than 30 minutes, as indicated at 906. In this way, if multiple MS events are detected and the case duration is greater than 30 minutes, the final MS event detected in the first 8 minutes of the case may be selected as the actual MS event and maintenance may be plotted to begin at that MS event. However, if no MS event is detected in the first 8 minutes of the case (and the case duration is greater than 30 minutes), the first MS event that occurred between the start and end of the case is used as the start of maintenance, as indicated at 908.

Further, as indicated at 910, adjusting the duration of maintenance may include, if no MS event is detected and the case duration is in a range of 6-10 minutes, indicating the maintenance phase started 3 minutes after CS. If a MS event is not detected, it is likely that maintenance did occur but was not detectable/did not fulfill the detection criteria. Thus, only the first 3 minutes are considered as induction and maintenance is added as starting 3 minutes after the case start. Further still, as indicated at 912, adjusting the duration of maintenance may include, if no MS event is detected and the case duration is greater than 10 minutes, indicating the maintenance phase started 5 minutes after CS.

At 914, method 900 includes, if indicated, adjusting the duration of emergence. The duration of emergence may be adjusted due to more than one ES event being detected, due to emergence lasting longer than a threshold duration, or due to no ES events being detected. Adjusting the duration of emergence may include, as indicated at 916, if the case duration is greater than 30 minutes, using the first ES detected in the last 15 minutes of the case. In this way, if more than one ES event is detected, when the case duration is greater than 30 minutes, the first ES event that occurred in the final 15 minutes of the case may be set as the actual ES event and emergence may be plotted starting at that ES event. As indicated at 918, if no ES event is detected in the last 15 minutes of the case (and the case duration is greater than 30 minutes), the last ES event that occurred between the start and end of the case may be set as the actual ES event and emergence may be plotted starting at that ES event. Additionally, as indicated at 920, if the set ES event is determined to have occurred before the designated MS event, the set ES event is removed as being invalid. Further, adjusting the duration of emergence may include, as indicated at 922, shortening the duration of emergence if emergence is greater than 30 minutes long. For example, the duration of emergence may be cut to the last 10 minutes of the case. As such, the ES event may be moved from the detected/set ES event to be 10 minutes before CE. Emergence typically does not last longer than 30 minutes. In some examples, emergence may be prolonged due to the anesthesia machine being kept on while the patient is removed. Further, false emergence detections can occur at the beginning of the case and the actual emergence may be missed in such examples, leading to longer emergence than actually occurred. By shortening the duration of emergence when emergence is longer than 30 minutes, emergence may be reflected accurately based on the most likely scenarios. Further still, as indicated at 924, if no ES events are detected, the time that administration of anesthetic agent ended (time of last agent end, which may be determined based on the set or measured inspired anesthetic agent going to zero) may be set as the ES event. As explained above, if this set ES event is before the set MS event, the ES event may be removed.

At 926, method 900 includes setting the CE to the first CE detected. In this way, if multiple CE events are detected, the first CE may be set as the valid CE event and the case marked complete at the set/valid CE event. Method 900 then ends.

Thus, the systems and methods disclosed herein provide for anesthesia event detection and notification of patient anesthesia phase based on the anesthesia event detection. As described above with respect to FIGS. 8A-8C and shown in the examples illustrated in FIGS. 10-13 and described below, a GUI may be displayed that includes a first visual representation of a default or previously-determined phase of anesthesia delivery for a patient, such as in the form of a timeline. For example, the default phase may be "anesthesia delivery not started," which may be depicted via blank/white space on the timeline, or the previously-determined phase may be induction, which may be depicted via a first color or pattern on the timeline. A plurality of monitoring parameters of the patient may be received over an observation window, with at least a portion of the plurality of monitoring parameters being obtained from an anesthesia delivery machine. A set of rules may be applied to the plurality of monitoring parameters in order to identify whether an event signaling a change to a new phase of anesthesia delivery for the patient is detected, wherein the event is one of case start, maintenance start, emergence start, and case end. If the event is detected, the GUI may be updated to display a second visual representation of the new phase, such as changing a color or pattern on the timeline. Conversely, if the event is not detected, the first visual representation may be maintained on the GUI, for example, the timeline may continue to depict the color or pattern that indicates the default or previously-determined phase.

Further, as explained above with respect to FIG. 6, some of the received monitoring parameters may be received in a standardized format (e.g., at a first, higher frequency), while other monitoring parameters may be received in a non-standardized format (e.g., at a second, lower frequency). The monitoring parameters received in the non-standardized format may be converted to the standardized format (e.g., to the first frequency via interpolation with the prior 30 seconds' worth of values for that monitoring parameter) before the monitoring parameters are analyzed to detect an event as described above. In doing so, various different monitoring parameters indicative of anesthesia delivery steps/actions may be analyzed in the same standardized format, regardless of the format in which the monitoring parameters were received.

For example, the systems and methods disclosed herein may be configured to output, for display on a display device, a GUI including a first visual representation of a default or previously-determined phase of anesthesia delivery for the patient; receive a plurality of monitoring parameters of the patient over an observation window, at least a portion of the plurality of monitoring parameters obtained from an anesthesia delivery machine; identify, by applying a set of rules to the plurality of monitoring parameters, whether an event signaling a change to a new phase of anesthesia delivery for the patient is detected, wherein the event is one of case start, maintenance start, emergence start, and case end; based on the event being detected and determined to be a true event, update the GUI to display a second visual representation of the new phase; and based on the event not being detected, maintain the first visual representation on the GUI.

In some examples, applying the set of rules to identify whether the event is detected may include applying a first subset of rules to determine if case start is detected; applying a second subset of rules and a third subset of rules to determine if maintenance start is detected; applying a fourth subset of rules and a fifth subset of rules to determine if emergence start is detected; and applying a sixth subset of rules to determine if case end is detected. In some examples, applying the first subset of rules to determine if case start is detected comprises determining that case start is detected responsive to an end of a first standby event of the anesthesia delivery machine; applying the second subset of rules and the third subset of rules to determine if maintenance start is detected comprises determining that maintenance start is detected responsive to a first of: (a) and (b) being true or (c) being true, wherein (a) comprises a mechanical ventilation start event occurring, (b) comprises at least one valid value of end-tidal carbon dioxide (EtCO2), and (c) comprises a fresh gas flow pattern of: two initial fresh gas flow values being above a threshold, at least three out of the next five fresh gas flow values being below the threshold, and a third fresh gas flow value being below the threshold; applying the fourth subset of rules and the fifth subset of rules to determine if emergence start is detected comprises determining that emergence start is detected responsive to a first of: (d) and (e) being true or (f) being true, wherein (d) comprises a mechanical ventilation end event occurring and observed for a threshold amount of time, (e) comprises no cardiac bypass mode, and (f) comprises two of an increase in O2 percentage, an increase in fresh gas flow, and an end of anesthetic agent delivery being true; and applying the sixth subset of rules to determine if case end is detected comprises determining that case end is detected responsive to a start of a second standby event of the anesthesia delivery machine. In some examples, the second subset of rules and the third subset of rules are applied in parallel, and the fourth subset of rules and the fifth subset of rules are applied in parallel.

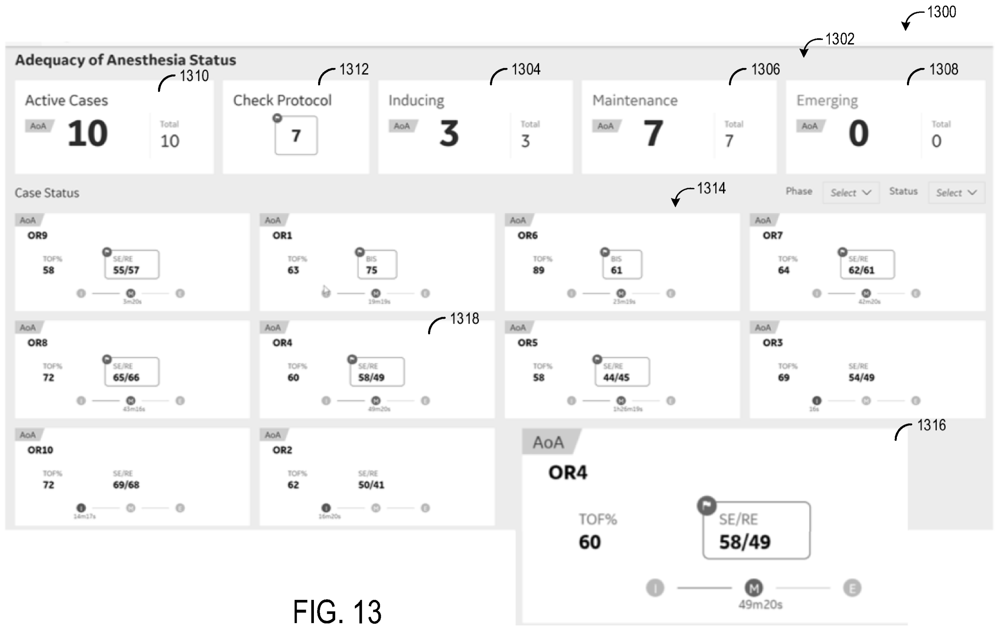

In some examples, the systems and methods disclosed herein further include if case start is detected, determining that the case start is a true event and updating the GUI to display the second visual representation, the second visual representation indicating that the patient is currently in induction, regardless of the previously-determined phase of anesthesia delivery; if maintenance start is detected, determining that the maintenance start is a true event and updating the GUI to display the second visual representation, the second visual representation indicating that the patient is currently in maintenance, regardless of the previously-determined phase of anesthesia delivery; if emergence start is detected during maintenance, determining that the emergence start is a true event and updating the GUI to display the second visual representation, the second visual representation indicating that the patient is currently in emergence; if emergence start is detected during induction, determining that the emergence start is not a true event, and maintaining the first visual representation on the GUI; if case end is detected, determining that the case end is a true event, and updating the GUI to display the second visual representation, the second visual representation indicating that the patient is currently in case end, regardless of the previously-determined phase of anesthesia delivery; and if none of case start, maintenance start, emergence start, and case end is detected, maintaining the first visual representation on the GUI. It is to be appreciated that the GUI may be the GUI shown in FIGS. 10-12 and/or the second GUI of FIG. 13.

In some examples, when the event is case end, and responsive to case end being detected, the case is marked as complete and a seventh subset of rules is applied to retrospectively update a timing and/or occurrence of one or more phases of anesthesia delivery for the case, as explained above with respect to FIG. 9. For example, applying the seventh subset of rules to retrospectively update a timing and/or occurrence of one or more phases of anesthesia delivery for the case may include: if more than one instance of a given type of event was detected during the case, selecting one instance of the given type of event as a valid event and updating the timing of a phase corresponding to the given type of event; and if no instance of a given type of event was detected during the case, adding the given type of event and updating the occurrence of the phase corresponding to the given type of event. In some examples, the given type of event is maintenance start, such that if more than one instance of maintenance start is detected during the case, one instance of maintenance start is selected as the valid event, where selecting the one instance of maintenance start comprises, if a duration of the case is greater than a threshold duration, selecting a last instance of maintenance start that occurred during a first time period of the case. In some examples, if no instance of maintenance start is detected during the case, adding maintenance start to the case comprises: if the duration of the case is within a threshold range, designating a first predefined amount of time since case start as maintenance; and if the duration of the case is greater than the threshold range, designating a second predefined amount of time since case start as maintenance, the second predefined amount of time greater than the first predefined amount of time.

In some examples, the given type of event is emergence start, such that if more than one instance of emergence start is detected during the case, one instance of emergence start is selected as the valid event, where selecting the one instance of emergence start comprises: if a duration of the case is greater than a threshold duration, selecting a first instance of emergence start that occurred during a last time period of the case; and if the duration of the case is greater than the threshold duration and no instances of emergence start were detected in the last time period of the case, selecting the last instance of emergence start detected during the case. In some examples, if no instance of emergence start is detected during the case, adding emergence start to the case comprises determining a time at which delivery of anesthetic agent to the patient ended and adding emergence start at the determined time.

The systems and methods disclosed herein may detect multiple events for a given patient (e.g., a first event and a second event) and determine, based on the first event and the second event, if the first event or the second event is a false positive event. If the first event or the second event is a false positive event, the GUI may be updated to reflect the false positive event (e.g., the timeline may be adjusted to remove the false positive event. For example, the first event may be emergence start and the second event may be maintenance start, wherein determining, based on the first event and the second event, that the first event or the second event is a false positive event comprises determining that the emergence start is the false positive event based on the maintenance start being detected after the emergence start was detected. In such examples, the GUI may be updated by removing emergence from the timeline for the patient and extending a duration of maintenance on the timeline. As another example, the first event may be maintenance start and the second event may be case start, wherein determining, based on the first event and the second event, that the first event or the second event is a false positive event comprises determining that the maintenance start is the false positive event based on the case start being detected after the maintenance start was detected. In such examples, the GUI may be updated by removing maintenance from the timeline for the patient and plotting induction on the timeline.

FIG. 10 shows an example GUI 1000 in a first state 1001. GUI 1000 may be displayed on a display device (e.g., of first care provider device 134) and may be generated and updated according to method 800 of FIGS. 8A-8C and method 900 of FIG. 9 based on anesthesia delivery event detections according to methods 600 and 700 of FIGS. 6 and 7. GUI 1000 may include an OR status panel 1002 that includes a plurality of display elements that provide a summary of current phases for all of a plurality of patients undergoing anesthesia delivery at a specific facility, ward, or unit (e.g., the operating rooms of a hospital). The plurality of display elements may include a first display element 1004 that indicates the number of patients undergoing induction, a second display element 1006 that indicates the number of patients undergoing maintenance, and a third display element 1008 that indicates the number of patients undergoing emergence. Additional display elements may be included in the OR status panel, such as a fourth display panel that indicates the total number of patients in a monitoring mode (e.g., where the anesthesia delivery machine has been switched to a "monitoring only mode") and/or the number of open operating rooms.

GUI 1000 further includes a plurality of anesthesia delivery timelines 1010. Each timeline of the plurality of timelines 1010 depicts anesthesia delivery phase as a function of time for a respective patient of the plurality of patients. In some examples, the amount of time that is shown on the GUI 1000 may be adjusted by a user by selecting a time element. As shown in FIG. 10, a user has selected to view the past three hours of time and the timelines are adjusted to show three hours of time. The user may instead select to see the past hour of time, the past six hours of time, the past day (e.g., 12 or 24 hours), or another suitable amount, and the timelines may be expanded or contracted accordingly.

The timelines may be arranged by operating room or anesthesia machine. For example, a first row 1011 is depicted that shows anesthesia delivery phase as a function of time for the patient(s) within a selected operating room, in this example OR4. The first row 1011 includes timelines for three different patients (e.g., three different cases), two of which have already been completed (such as completed timeline 1013) and one that is active, shown as active timeline 1012. The active timeline 1012 may visually depict current anesthesia phase as a color-coded bar. For example, induction is shown as a first color at region 1014 and maintenance is shown as a second color at region 1016. Induction (e.g., region 1014) may commence at the time specified in a first indication that case start was detected for the patient. Maintenance may commence at the time specified in a second indication that maintenance start was detected for the patient. The first and second indications may be generated as described above with respect to FIGS. 6-8C. Thus, when the second indication is received (indicating detection of an event signaling a change to a new phase), the active timeline 1012 may be adjusted to add the new phase (e.g., changing from the first color to the second color). After the second indication is received, additional indications received may not identify an event and thus the active timeline 1012 may be maintained (e.g., the region 1016 may extend in time but stay in the second color).

In some examples, GUI 1000 may further include a pop-up panel 1018 that may be displayed in response to a hover input being received over a selected phase of a selected timeline (e.g., an input being received to region 1014 of timeline 1012). The pop-up panel 1018 may include additional information about the selected phase, such as the phase name (e.g., induction), duration of the phase, start time of the phase, and end time of the phase. In the example shown, the pop-up panel 1018 indicates that induction started at 9:25, ended at 9:35, and lasted 10 minutes.

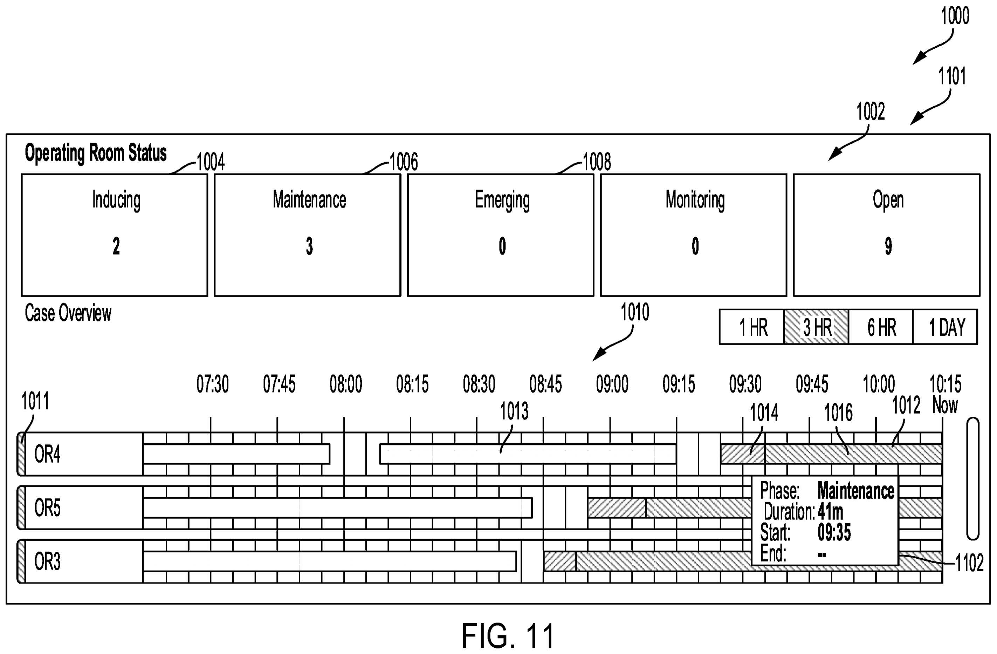

GUI 1000 is shown in a second state 1101 in FIG. 11. In the second state, a different hover input has been received (e.g., over region 1016 of active timeline 1012), resulting in another pop-up panel 1102 being displayed that identifies maintenance phase started at 9:35, has yet to end (e.g., is ongoing), and has a current duration of 41 minutes.

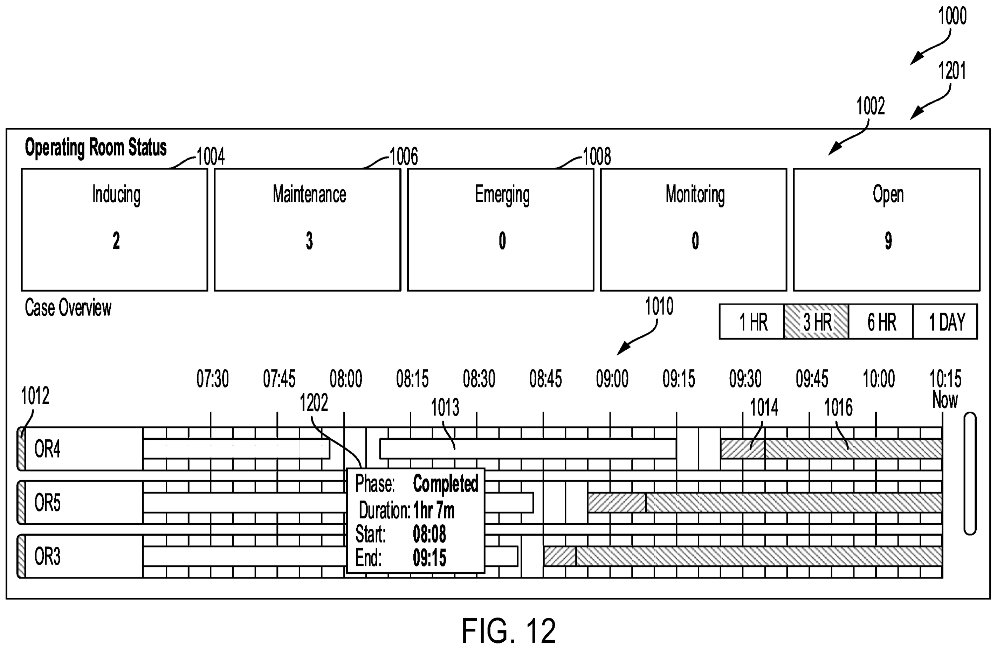

GUI 1000 is shown in a third state 1201 in FIG. 12. In the third state, a different hover input has been received (e.g., over completed timeline 1013), resulting in another pop-up panel 1202 being displayed that identifies the prior case occurring in OR4 (e.g., a prior patient) has completed, with a duration of 1 hour 7 minutes, starting at 8:08 and ending at 9:15. The completed case may be identified with a particular color, such as gray. While not shown in FIG. 12, it is to be appreciated that in some examples, the completed timeline 1013 may visually depict the various phases determined for that case (e.g., by color, with text, or another suitable visual representation).

As explained above with respect to FIGS. 8A-9, the active timelines and/or the completed timelines may be updated if it is determined an initial detected event is not the actual/valid event. For example, a first CS event being detected (e.g., at 9:24) may trigger plotting of induction on the active timeline 1012. If a second CS event is detected after the first CS event, the first CS event may be discarded and the active timeline 1012 may be adjusted to show that induction started upon the second CS event (e.g., at 9:25). The first MS event detected may trigger the switch to plotting maintenance on the active timeline 1012, and any additional MS events may not affect the active timeline 1012. As the case progresses, an ES event may be detected, which would trigger a switch to plotting emergence on the active timeline 1012 (not shown, but it is to be understood that emergence may be shown in a third color). However, if a MS event is detected while emergence is being plotted, the case may revert back to maintenance. In such an example, the portion of the active timeline 1012 that was originally plotted in the third color to show emergence may be switched to the second color to show maintenance. Similarly, once the case is marked complete and the active timeline 1012 switches to a completed timeline, aspects of the timeline may be adjusted as explained above with respect to FIG. 9 if multiple CS events, MS events, ES events, and/or CE events were detected, if no MS or ES event was detected, etc.

In this way, both the real-time/active visual representation of anesthesia phases for a patient and the completed visual representation of anesthesia phases for the patient may be adjusted when indicated to account for false positive event detections (e.g., when a particular event is detected more than once during a case) and false negative event detections (e.g., when no events for a particular phase are detected during a case). For example, in certain use cases, a clinician may turn on and turn off the mechanical ventilator at the very beginning of the case in order to test and confirm the ventilator is working properly. Activation of the ventilator may indicate that the current phase is maintenance, while subsequent deactivation of the ventilator may indicate that the current phase is emergence. To reduce false positive emergence detection, the case may be determined to in emergence only if the ventilator stays off for at least a threshold amount of time (e.g., 55 seconds). If the ventilator does stay off the threshold amount of time and then a second activation of the ventilator is detected, the system may assume the second activation is the actual entry into maintenance and update the GUI accordingly. Further, there could be situations where emergence is detected, but the case is still in maintenance, which could cause false detections. Thus, after an ES event is detected and then a MS event is detected, the system may assume the ES event was a false positive event and revert the case back to maintenance. Situations with multiple ventilation switches back and forth also provides signals for nondeterministic event detection, and thus, as explained above, the system may assume the case is in maintenance each time the ventilator is turned on, if the anesthesia delivery machine is maintained active (e.g., not put into standby mode, which would signal that the case is complete).

While not shown in the GUI of FIGS. 10-12, in some examples, various notifications may be provided via the GUI, such as notifications that indicate a particular phase of anesthesia delivery for a patient has extended beyond a threshold duration. For example, if induction for the patient lasts longer than a first threshold duration (e.g., ten minutes), a first notification may be displayed to alert users of the extended induction phase, as the extended case start phase may indicate a delay in progressing the patient to reception of anesthesia due to unavailability of equipment and/or personnel. As another example, if emergence for the patient lasts longer than a second threshold duration (e.g., 20 minutes), a second notification may be displayed to alert uses of the extended emergence, which may indicate that the patient is exhibiting deep anesthesia and/or that logistical issues (e.g., lack of beds or personnel) are hindering movement of the patient out of the operating room. By automatically determining anesthesia phase for each of a plurality of patients, visually indicating the current phase for each patient via the GUI, and notifying when a current phase has extended beyond a threshold duration, a user may be apprised of the current status and/or any issues arising with anesthesia delivery for each patient without having to be in the room with the patient, without having to directly communicate with any clinicians attending to the patient, and without having to access an electronic medical records or other data sources pertaining to the patients. In doing so, efficiency of the overall system may be improved by reducing undue delays between patients in the operating rooms, reducing user interaction with patient data/monitoring parameters in an attempt to ascertain the current phase of anesthesia delivery for one or more or each patient in the medical facility, and the like.

In still further examples, one or more notifications may be displayed when a pause or interruption in reception of one or more monitoring parameters for a patient occurs. As explained above, the events that signal a change to a new phase of anesthesia delivery may only occur during one observation window, or over a few observation windows. As such, the majority of indications that are generated may indicate that no event is detected and the GUI may continue to maintain the previously-determined anesthesia phase. However, if one or more monitoring parameters for a patient are not received for a period of time (e.g., the anesthesia delivery machine for that patient loses connection to the network), the GUI may continue to indicate that the patient is in the previous anesthesia phase even if the patient has transitioned to a new phase, as the event signaling the change to the new phase may be missed due to the monitoring parameters not being received during the event. As such, if one or more monitoring parameters are not received over the observation window or over two or more consecutive observation windows, an indication may be generated that the event detection (or lack thereof) is not reliable and/or rather than sending an indication of a detected event or an indication that no event was detected, an indication may be sent that indicates insufficient monitoring parameter data was received to determine if an event was detected or not. In such examples, the GUI may be updated to reflect the unreliable or undeterminable event/no event detection.

As explained previously, the detected phases of anesthesia may be used in various algorithms, such as adequacy of anesthesia. FIG. 13 shows an example second GUI 1300 that indicates the output of an adequacy of anesthesia (AoA) algorithm that utilizes detected anesthesia phase. The second GUI includes a status panel 1302 that is similar to the OR status panel 1002 of the GUI 1000, and thus includes a first display element 1304 that indicates the number of patients undergoing induction, a second display element 1306 that indicates the number of patients undergoing maintenance, and a third display element 1308 that indicates the number of patients undergoing emergence. Additional display elements may be included in the status panel 1302, such as a fourth display element 1310 that indicates the total number of active cases and a fifth display element 1312 that indicates the number of cases where the AoA algorithm has triggered an alert/flag to check the protocol for anesthesia delivery. The protocol for anesthesia delivery may dictate, among other parameters, the type of anesthesia to be delivered and optimal ranges for various anesthesia parameters, referred to as AoA parameters, which may be based on the current phase of anesthesia. The protocol for anesthesia delivery may be set/selected by a user (e.g., anesthesiologist) and may be specific to each patient, general for all patients, or a combination (e.g., a first protocol may be applied to adult patients while a second protocol may be applied to pediatric patients).

The second GUI further includes a plurality of AoA display panels 1314 that each include AoA parameters for each active patient/case, such as neuromuscular transmission monitoring parameters (e.g., TOF%), bispectral index (BIS), and/or entropy monitoring parameters (e.g., state entropy (SE)/response entropy (RE)), as well as the current anesthesia phase for each case (e.g., induction, maintenance, or emergence) and the duration of the current phase. FIG. 13 includes a magnified view 1316 of an AoA display panel 1318. As shown in the magnified view 1316, the case/patient (e.g., in OR4) is currently in maintenance and has been in maintenance for 49 minutes and 20 seconds. The current phase and duration in the current phase may be determined as described above with respect to FIGS. 6 and 7 and used to update the second GUI 1300 as explained above with respect to FIGS. 8A-8C. Further, the magnified view 1316 shows that the patient has an SE/RE ratio that has triggered an alert/flag. In some examples, the alert/flag may be based on the current anesthesia phase. For example, during induction, SE may fall to an optimal range (e.g., 40-60) and may be less than RE, which may indicate sufficient depth of anesthesia but not deep anesthesia (that is to be avoided). During maintenance, the SE and RE should be equal or within a narrow range of each other. During emergence, it may be optimal for SE to be above 65. If the values of SE and/or RE deviate from these rules, an alert/flag may be triggered. In the example shown in FIG. 13, in the magnified view 1316, SE is higher than RE during maintenance and thus has triggered the alert/flag. As such, to know if the SE/RE values are optimal/meet the rules of the AoA protocol, the current phase of anesthesia is needed. The methods described herein may be used to ensure real-time, accurate detection of the current phase of anesthesia for each case/patient in the medical facility, which can be displayed to one or more clinicians via the GUIs disclosed herein and used to accurately determine if the anesthesia delivery is sufficient (and not too high) for each phase.

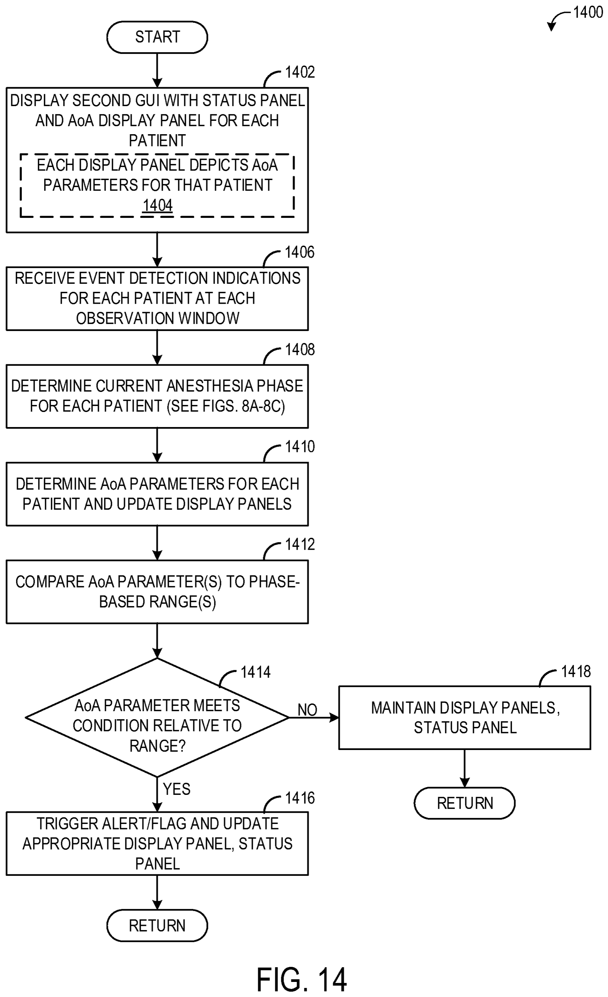

FIG. 14 is flow chart illustrating a method 1400 for updating a second GUI, such as second GUI 1300. Method 1400 may be implemented with the system 10 of FIGS. 1 and 2. Method 1400 may be carried out according to instructions stored in non-transitory memory and executed by one or more processors, such as memory and processor(s) of computing system 120 of FIG. 2. In some examples, method 1400 may instead be carried out according to instructions stored in non-transitory memory and executed by one or more processors of a computing system in communication with computing system 120 of FIG. 2, such as a care provider device (e.g., first care provider device 134). In some examples, method 1400 may be carried out as part of method 600, e.g., at 617 of method 600, and in combination with method 800.

At 1402, method 1400 includes displaying a second GUI including a status panel and an AoA display panel for each patient currently undergoing anesthesia delivery. In some examples, rather than identifying the patients themselves, each AoA display panel may be specific to a respective operating room or each anesthesia delivery machine that is in operation as a proxy for identifying a patient. The second GUI may be the second GUI 1300 and thus the status panel may include a first display element that indicates the number of patients undergoing induction, a second display element that indicates the number of patients undergoing maintenance, a third display element that indicates the number of patients undergoing emergence, a fourth display element that indicates the total number of active cases, and a fifth display element that indicates the number of cases where the AoA algorithm has triggered an alert/flag to check the protocol for anesthesia delivery, which will be explained in more detail below. Each AoA display panel depicts AoA parameters for a corresponding patient, as indicated at 1404. The AoA parameters may include, for each patient, the most-recently determined TOF%, SE/RE values, and/or BIS value, as well as the current phase of anesthesia delivery and duration in the current phase. The current phase of anesthesia delivery may be determined according to method 800, as described above.

At 1406, method 1400 includes receiving an event detection indication for each patient at each observation window. The indications may be generated according to method 600 and method 700, for example. As explained above, an event may include one of case start, maintenance start, emergence start, and case end. The method to identify if an event has occurred (e.g., method 600 and method 700) may be executed for each patient that is about to start receiving anesthesia or is currently receiving anesthesia. As explained above with respect to FIG. 6, during each observation window, for each patient (or operating room or anesthesia delivery machine), an indication may be generated of whether an event has been detected for that patient or whether an event has not been detected for that patient. The indications may be stored and/or used to determine a current anesthesia phase for each, as indicated at 1408, and as explained above with respect to FIGS. 8A-8C.

At 1410, the AoA parameters are determined for each patient and, when indicated, the AoA display panels are updated to reflect any changing AoA parameters. As an example, when a case start event is detected for a patient, a new AoA display panel may be added to the second GUI for that patient, and the AoA display panel may show that the current phase is induction and display a TOF% and SE/RE values or a BIS value for that patient. As another example, if calculated SE/RE values change for a patient, the AoA panel for that patient may be updated to display the new SE/RE values.

At 1412, one or more of the AoA parameters may be compared to a corresponding range. In some examples, the corresponding range may be a phase-based range. For example, a BIS value for a patient may be compared to a first BIS range, wherein the first BIS range is selected based on the current anesthesia phase for the patient. As another example, SE/RE values (or ratio) for a patient may be compared to first SE/RE ranges (or a first SE/RE ratio range), wherein the first SE/RE ranges or ratio range are/is selected based on the current anesthesia phase for the patient.

At 1414, method 1400 includes determining if any AoA parameter meets a condition relative to a corresponding range. For example, method 1400 may include determining if any BIS values are greater than, or less than, a corresponding BIS range. As a non-limiting example, a BIS range for a first patient may be 40-60 and if the BIS value for the first patient is greater than 60 or less than 40, the BIS value may be determined to meet the condition relative to the BIS range.

If an AoA parameter does meet the condition relative to the corresponding range, method 1400 proceeds to 1416 to trigger an alert/flag and update the appropriate AoA display panel as well as the status panel of the second GUI. For example, using the above scenario where the BIS value for the first patient is above or below the BIS range for the first patient, the AoA display panel for the first patient may be updated to visually indicate the alert/flag. Further, the fifth display element of the status panel may be updated to reflect the alert/flag for the first patient (e.g., the fifth display element may display a total count of patients for which an alert/flag has been triggered). Method 1400 then returns, such that the method 1400 may be repeated at a given frequency (e.g., once a second) to provide real-time/live updates to each patient's AoA parameters. It is to be appreciated that once an alert/flag has been triggered for a patient, the AoA display panel for that patient may continue to display the visual indication of the alert/flag until it is determined that the AoA parameter for the patient no longer meets the condition relative to the corresponding rage. For example, a clinician may adjust the amount of anesthesia delivered to the patient and as a result, the BIS value for the patient may fall into the corresponding range, at which point the alert/flag may be removed.

Returning to 1414, if no AoA parameters meet a condition relative to a corresponding range, method 1400 proceeds to 1418 to maintain the AoA display panels and status panel, and then method 1400 returns, such that the method 1400 may be repeated at a given frequency (e.g., once a second) to provide real-time/live updates to each patient's AoA parameters.

The systems and methods described herein provide specific technical improvements to computer technology and medical monitoring systems in several ways. First, the systems disclosed herein improve the technical field of anesthesia monitoring by providing an automated solution to complex technical challenges that cannot practically be performed mentally. Specifically, the systems disclosed herein analyze multiple real-time physiological parameters simultaneously and apply specialized rule-based algorithms in parallel to detect key transition events with high temporal precision. This parallel processing approach enables the systems disclosed herein to identify phase transitions that would be impractical to calculate manually given the volume and frequency of the monitoring data.

Second, the systems disclosed herein provide technological improvements through specialized processing architecture, which includes a stateless design that processes each observation window independently, reducing memory overhead and improving system reliability by preventing error propagation; parallel application of multiple rule subsets to enable simultaneous evaluation of different phase transition criteria; real-time analysis of time-series medical device data using sliding windows with configurable durations and frequencies; dynamic interpolation capabilities to handle variable input frequencies from different monitoring parameters; and automated retrospective analysis to detect and correct false positive and false negative event detections.

Third, the systems disclosed herein implement specific technical solutions to form a specifically configured system, including rule-based logic engines that process multiple physiological parameters simultaneously to detect phase transitions; specialized algorithms for handling edge cases like cardiac bypass and total intravenous anesthesia; automated phase detection that functions across different anesthesia machine configurations without requiring explicit clinician input; and real-time processing of streaming medical device data to enable immediate phase detection and GUI updates.

A technical effect of identifying, by applying a selected set of rules to a plurality of patient monitoring parameters, whether an event signaling a change to a new phase of anesthesia delivery for a patient is detected, and if the event is detected, updating a GUI to display a visual representation of the new phase, is that anesthesia phase may be detected automatically using parameters from the anesthesia delivery machine without requiring separate/dedicated monitoring devices such as EEGs, which may increase processing efficiency by utilizing parameters already available/being analyzed and without having to process and analyze data from the separate/dedicated monitoring devices. The anesthesia phase may be displayed or otherwise indicated to one or more users, such as administrators or clinicians, which may facilitate enhanced patient care and operational efficiency. By utilizing the event detection described herein, events signaling a change to a new phase of anesthesia delivery may be detected as quickly as the available monitoring parameters allow without having to know the specific configuration of the particular anesthesia delivery machine being used. Further, by using a sliding observation window of monitoring parameters (that is the same duration for each parameter and each observation window) of a suitable duration (e.g., one minute), slow/delayed parameters may still be detected while allowing for rapid detection of an event.

The disclosure also provides support for a system for tracking phases of anesthesia delivery over a case for a patient, comprising: one or more processors, and memory storing instructions executable by the one or more processors to: output, for display on a display device, a graphical user interface (GUI) including a first visual representation of a default or previously-determined phase of anesthesia delivery for the patient, receive a plurality of monitoring parameters of the patient over an observation window, at least a portion of the plurality of monitoring parameters obtained from an anesthesia delivery machine, identify, by applying a set of rules to the plurality of monitoring parameters, whether an event signaling a change to a new phase of anesthesia delivery for the patient is detected, wherein the event is one of case start, maintenance start, emergence start, and case end, based on the event being detected and determined to be a true event, update the GUI to display a second visual representation of the new phase, and based on the event not being detected, maintain the first visual representation on the GUI. In a first example of the system, executing the instructions to apply the set of rules to identify whether the event is detected comprises: applying a first subset of rules to determine if case start is detected, applying a second subset of rules and a third subset of rules to determine if maintenance start is detected, applying a fourth subset of rules and a fifth subset of rules to determine if emergence start is detected, and applying a sixth subset of rules to determine if case end is detected. In a second example of the system, optionally including the first example,: applying the first subset of rules to determine if case start is detected comprises determining that case start is detected responsive to an end of a first standby event of the anesthesia delivery machine, applying the second subset of rules and the third subset of rules to determine if maintenance start is detected comprises determining that maintenance start is detected responsive to a first of: (a) and (b) being true or (c) being true, wherein (a) comprises a mechanical ventilation start event occurring, (b) comprises at least one valid value of end-tidal carbon dioxide (EtCO2), and (c) comprises a fresh gas flow pattern of: two initial fresh gas flow values being above a threshold, at least three out of the next five fresh gas flow values being below the threshold, and a third fresh gas flow value being below the threshold, applying the fourth subset of rules and the fifth subset of rules to determine if emergence start is detected comprises determining that emergence start is detected responsive to a first of: (d) and (e) being true or (f) being true, wherein (d) comprises a mechanical ventilation end event occurring and observed for a threshold amount of time, (e) comprises no cardiac bypass mode, and (f) comprises two of an increase in O2 percentage, an increase in fresh gas flow, and an end of anesthetic agent delivery being true, and applying the sixth subset of rules to determine if case end is detected comprises determining that case end is detected responsive to a start of a second standby event of the anesthesia delivery machine. In a third example of the system, optionally including one or both of the first and second examples, the second subset of rules and the third subset of rules are applied in parallel, and wherein the fourth subset of rules and the fifth subset of rules are applied in parallel. In a fourth example of the system, optionally including one or more or each of the first through third examples, updating the GUI to display the second visual representation of the new phase based on the event being detected and determined to be a true event comprises: if case start is detected, determining that the case start is a true event and updating the GUI to display the second visual representation, the second visual representation indicating that the patient is currently in induction, regardless of the previously-determined phase of anesthesia delivery, if maintenance start is detected, determining that the maintenance start is a true event and updating the GUI to display the second visual representation, the second visual representation indicating that the patient is currently in maintenance, regardless of the previously-determined phase of anesthesia delivery, if emergence start is detected during maintenance, determining that the emergence start is a true event and updating the GUI to display the second visual representation, the second visual representation indicating that the patient is currently in emergence, if emergence start is detected during induction, determining that the emergence start is not a true event, and maintaining the first visual representation on the GUI, if case end is detected, determining that the case end is a true event, and updating the GUI to display the second visual representation, the second visual representation indicating that the patient is currently in case end, regardless of the previously-determined phase of anesthesia delivery, and if none of case start, maintenance start, emergence start, and case end is detected, maintaining the first visual representation on the GUI. In a fifth example of the system, optionally including one or more or each of the first through fourth examples, the event is case end, and responsive to case end being detected, marking the case as complete and applying a seventh subset of rules to retrospectively update a timing and/or occurrence of one or more phases of anesthesia delivery for the case. In a sixth example of the system, optionally including one or more or each of the first through fifth examples, applying the seventh subset of rules to retrospectively update a timing and/or occurrence of one or more phases of anesthesia delivery for the case comprises: if more than one instance of a given type of event was detected during the case, selecting one instance of the given type of event as a valid event and updating the timing of a phase corresponding to the given type of event, and if no instance of a given type of event was detected during the case, adding the given type of event and updating the occurrence of the phase corresponding to the given type of event. In a seventh example of the system, optionally including one or more or each of the first through sixth examples, the given type of event is maintenance start, such that if more than one instance of maintenance start is detected during the case, one instance of maintenance start is selected as the valid event, where selecting the one instance of maintenance start comprises, if a duration of the case is greater than a threshold duration, selecting a last instance of maintenance start that occurred during a first time period of the case. In an eighth example of the system, optionally including one or more or each of the first through seventh examples, if no instance of maintenance start is detected during the case, adding maintenance start to the case comprises: if the duration of the case is within a threshold range, designating a first predefined amount of time since case start as maintenance, and if the duration of the case is greater than the threshold range, designating a second predefined amount of time since case start as maintenance, the second predefined amount of time greater than the first predefined amount of time. In a ninth example of the system, optionally including one or more or each of the first through eighth examples, the given type of event is emergence start, such that if more than one instance of emergence start is detected during the case, one instance of emergence start is selected as the valid event, where selecting the one instance of emergence start comprises: if a duration of the case is greater than a threshold duration, selecting a first instance of emergence start that occurred during a last time period of the case, and if the duration of the case is greater than the threshold duration and no instances of emergence start were detected in the last time period of the case, selecting the last instance of emergence start detected during the case. In a tenth example of the system, optionally including one or more or each of the first through ninth examples, if no instance of emergence start is detected during the case, adding emergence start to the case comprises determining a time at which delivery of anesthetic agent to the patient ended and adding emergence start at the determined time.

The disclosure also provides support for a system, comprising: one or more processors, and memory storing instructions executable by the one or more processors to: output, for display on a display device, a graphical user interface (GUI) including a plurality of timelines of anesthesia delivery phases, each timeline depicting anesthesia delivery phase as a function of time for a respective patient of a plurality of patients, for a first patient of the plurality of patients, receive a plurality of monitoring parameters of the first patient over a first observation window, at least a portion of the plurality of monitoring parameters obtained from an anesthesia delivery machine, identify, in real-time and without knowing a current or prior phase of anesthesia delivery for the first patient, that a first event signaling a change to a new phase of anesthesia delivery for the first patient is detected based on the plurality of monitoring parameters over the first observation window, wherein the first event is one of case start, maintenance start, emergence start, and case end, based on the first event being detected, update the GUI to add the new phase to a first timeline of the plurality of timelines, the first timeline representing anesthesia delivery phases for the first patient, identify that a second event for the patient is detected based on the plurality of monitoring parameters of the first patient over a second observation window, determine, based on the first event and the second event, if the first event or the second event is a false positive event, if the first event or the second event is a false positive event, update the GUI to adjust the first timeline to reflect the false positive event, and if the first event or the second event is not a false positive event, update the GUI to add a further new phase to the first timeline. In a first example of the system, executing the instructions to identify that the first event and/or second event is detected comprises: applying a first subset of rules to determine if case start is detected, applying a second subset of rules and a third subset of rules to determine if maintenance start is detected, applying a fourth subset of rules and a fifth subset of rules to determine if emergence start is detected, and applying a sixth subset of rules to determine if case end is detected. In a second example of the system, optionally including the first example,: applying the first subset of rules to determine if case start is detected comprises determining that case start is detected responsive to an end of a first standby event of the anesthesia delivery machine, applying the second subset of rules and the third subset of rules to determine if maintenance start is detected comprises determining that maintenance start is detected responsive to a first of: (a) and (b) being true or (c) being true, wherein (a) comprises a mechanical ventilation start event occurring, (b) comprises at least one valid value of end-tidal carbon dioxide (EtCO2), and (c) comprises a fresh gas flow pattern of: two initial fresh gas flow values being above a threshold, at least three out of the next five fresh gas flow values being below the threshold, and a third fresh gas flow value being below the threshold, applying the fourth subset of rules and the fifth subset of rules to determine if emergence start is detected comprises determining that emergence start is detected responsive to a first of: (d) and (e) being true or (f) being true, wherein (d) comprises a mechanical ventilation end event occurring and observed for a threshold amount of time, (e) comprises no cardiac bypass mode, and (f) comprises two of an increase in O2 percentage, an increase in fresh gas flow, and an end of anesthetic agent delivery being true, and applying the sixth subset of rules to determine if case end is detected comprises determining that case end is detected responsive to a start of a second standby event of the anesthesia delivery machine.

The disclosure also provides support for a method, comprising: outputting, for display on a display device, a graphical user interface (GUI) including a plurality of timelines of anesthesia delivery phases, each timeline depicting anesthesia delivery phase as a function of time for a respective patient of a plurality of patients, for a first patient of the plurality of patients, receiving a plurality of monitoring parameters of the first patient over a first observation window, at least a portion of the plurality of monitoring parameters obtained from an anesthesia delivery machine, identifying, in real-time and without knowing a current or prior phase of anesthesia delivery for the first patient, that a first event signaling a change to a new phase of anesthesia delivery for the first patient is detected based on the plurality of monitoring parameters over the first observation window, wherein the first event is one of case start, maintenance start, emergence start, and case end, based on the first event being detected, updating the GUI to add the new phase to a first timeline of the plurality of timelines, the first timeline representing anesthesia delivery phases for the first patient, identifying that a second event for the first patient is detected based on the plurality of monitoring parameters of the first patient over a second observation window, determining, based on the first event and the second event, that the first event or the second event is a false positive event, and in response, updating the GUI to adjust the first timeline to reflect the false positive event. In a first example of the method, the first event is emergence start, the new phase is emergence, and the second event is maintenance start, wherein determining, based on the first event and the second event, that the first event or the second event is a false positive event comprises determining that the emergence start is the false positive event based on the maintenance start being detected after the emergence start was detected, and wherein updating the GUI to adjust the first timeline to reflect the false positive event comprises removing emergence from the first timeline and extending a duration of maintenance on the first timeline. In a second example of the method, optionally including the first example, the first event is maintenance start, the new phase is maintenance, and the second event is case start, wherein determining, based on the first event and the second event, that the first event or the second event is a false positive event comprises determining that the maintenance start is the false positive event based on the case start being detected after the maintenance start was detected, and wherein updating the GUI to adjust the first timeline to reflect the false positive event comprises removing maintenance from the first timeline and plotting induction on the first timeline. In a third example of the method, optionally including one or both of the first and second examples, identifying that the first event is detected comprises: applying a first subset of rules to determine if case start is detected responsive to an end of a first standby event of the anesthesia delivery machine, applying a second subset of rules and a third subset of rules in parallel to determine if maintenance start is detected, applying a fourth subset of rules and a fifth subset of rules in parallel to determine if emergence start is detected, and applying a sixth subset of rules to determine if case end is detected responsive to a start of a second standby event of the anesthesia delivery machine. In a fourth example of the method, optionally including one or more or each of the first through third examples, the plurality of monitoring parameters comprises one or more of: measured fraction of inspired oxygen (FiO2), measured end-tidal CO2 (EtCO2), set mechanical ventilation, measured inspired anesthetic agent, measured expired anesthetic agent, fresh gas flow rate, cardiac bypass mode, and anesthesia machine standby state. In a fifth example of the method, optionally including one or more or each of the first through fourth examples, the GUI comprises: an operating room status panel indicating a total number of patients in each phase of anesthesia delivery, and wherein each timeline of the plurality of timelines is color-coded to indicate different phases of anesthesia delivery, the different phases comprising induction, maintenance, and emergence.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A system for tracking phases of anesthesia delivery over a case for a patient, comprising:
one or more processors; and
memory storing instructions executable by the one or more processors to:
output (802), for display on a display device, a graphical user interface (GUI) including a first visual representation of a default or previously-determined phase of anesthesia delivery for the patient;
receive (602) a plurality of monitoring parameters of the patient over an observation window, at least a portion of the plurality of monitoring parameters obtained from an anesthesia delivery machine;
identify (606), by applying a set of rules to the plurality of monitoring parameters, whether an event signaling a change to a new phase of anesthesia delivery for the patient is detected, wherein the event is one of case start, maintenance start, emergence start, and case end;
based on the event being detected and determined to be a true event, update (617) the GUI to display a second visual representation of the new phase; and
based on the event not being detected, maintain (810) the first visual representation on the GUI.

2. The system of claim 1, wherein executing the instructions to apply the set of rules to identify whether the event is detected comprises:
applying (702) a first subset of rules to determine if case start is detected;
applying (704, 706) a second subset of rules and a third subset of rules to determine if maintenance start is detected;
applying (708, 710) a fourth subset of rules and a fifth subset of rules to determine if emergence start is detected; and
applying (712) a sixth subset of rules to determine if case end is detected.

3. The system of claim 2, wherein:
applying (702) the first subset of rules to determine if case start is detected comprises determining that case start is detected responsive to an end of a first standby event of the anesthesia delivery machine;
applying (704, 706) the second subset of rules and the third subset of rules to determine if maintenance start is detected comprises determining that maintenance start is detected responsive to a first of: (a) and (b) being true or (c) being true, wherein (a) comprises a mechanical ventilation start event occurring, (b) comprises at least one valid value of end-tidal carbon dioxide (EtCO2), and (c) comprises a fresh gas flow pattern of: two initial fresh gas flow values being above a threshold, at least three out of the next five fresh gas flow values being below the threshold, and a third fresh gas flow value being below the threshold;
applying (708, 710) the fourth subset of rules and the fifth subset of rules to determine if emergence start is detected comprises determining that emergence start is detected responsive to a first of: (d) and (e) being true or (f) being true, wherein (d) comprises a mechanical ventilation end event occurring and observed for a threshold amount of time, (e) comprises no cardiac bypass mode, and (f) comprises two of an increase in O2 percentage, an increase in fresh gas flow, and an end of anesthetic agent delivery being true; and
applying (712) the sixth subset of rules to determine if case end is detected comprises determining that case end is detected responsive to a start of a second standby event of the anesthesia delivery machine.

4. The system of claim 3, wherein the second subset of rules and the third subset of rules are applied in parallel, and wherein the fourth subset of rules and the fifth subset of rules are applied in parallel.

5. The system of claim 3, wherein updating the GUI to display the second visual representation of the new phase based on the event being detected and determined to be a true event comprises:
if case start is detected (808), determining that the case start is a true event and updating (812) the GUI to display the second visual representation, the second visual representation indicating that the patient is currently in induction, regardless of the previously-determined phase of anesthesia delivery;
if maintenance start is detected (814), determining that the maintenance start is a true event and updating (816) the GUI to display the second visual representation, the second visual representation indicating that the patient is currently in maintenance, regardless of the previously-determined phase of anesthesia delivery;
if emergence start is detected during maintenance (818, 820), determining that the emergence start is a true event and updating (822) the GUI to display the second visual representation, the second visual representation indicating that the patient is currently in emergence;
if emergence start is detected during induction (818, 820), determining that the emergence start is not a true event, and maintaining (828) the first visual representation on the GUI;
if case end is detected (840), determining that the case end is a true event, and updating (842) the GUI to display the second visual representation, the second visual representation indicating that the patient is currently in case end, regardless of the previously-determined phase of anesthesia delivery; and
if none of case start, maintenance start, emergence start, and case end is detected, maintaining (810) the first visual representation on the GUI.

6. The system of claim 3, wherein the event is case end, and responsive to case end being detected, marking the case as complete and applying (620) a seventh subset of rules to retrospectively update a timing and/or occurrence of one or more phases of anesthesia delivery for the case, wherein applying the seventh subset of rules to retrospectively update a timing and/or occurrence of one or more phases of anesthesia delivery for the case comprises:
if more than one instance of a given type of event was detected during the case, selecting one instance of the given type of event as a valid event and updating the timing of a phase corresponding to the given type of event; and
if no instance of a given type of event was detected during the case, adding the given type of event and updating the occurrence of the phase corresponding to the given type of event.

7. The system of claim 6, wherein the given type of event is maintenance start, such that if more than one instance of maintenance start is detected during the case, one instance of maintenance start is selected as the valid event, where selecting the one instance of maintenance start comprises, if a duration of the case is greater than a threshold duration, selecting (906) a last instance of maintenance start that occurred during a first time period of the case.

8. The system of claim 7, wherein if no instance of maintenance start is detected during the case, adding maintenance start to the case comprises:
if the duration of the case is within a threshold range, designating (910) a first predefined amount of time since case start as maintenance; and
if the duration of the case is greater than the threshold range, designating (912) a second predefined amount of time since case start as maintenance, the second predefined amount of time greater than the first predefined amount of time.

9. The system of claim 8, wherein the given type of event is emergence start, such that if more than one instance of emergence start is detected during the case, one instance of emergence start is selected as the valid event, where selecting the one instance of emergence start comprises:
if a duration of the case is greater than a threshold duration, selecting (916) a first instance of emergence start that occurred during a last time period of the case; and
if the duration of the case is greater than the threshold duration and no instances of emergence start were detected in the last time period of the case, selecting (918) the last instance of emergence start detected during the case.

10. The system of claim 9, wherein if no instance of emergence start is detected during the case, adding emergence start to the case comprises determining a time at which delivery of anesthetic agent to the patient ended and adding (924) emergence start at the determined time.

11. A method, comprising:
outputting (802), for display on a display device, a graphical user interface (GUI) (1000) including a plurality of timelines (1010) of anesthesia delivery phases, each timeline depicting anesthesia delivery phase as a function of time for a respective patient of a plurality of patients;
for a first patient of the plurality of patients, receiving (602) a plurality of monitoring parameters of the first patient over a first observation window, at least a portion of the plurality of monitoring parameters obtained from an anesthesia delivery machine;
identifying (606), in real-time and without knowing a current or prior phase of anesthesia delivery for the first patient, that a first event signaling a change to a new phase of anesthesia delivery for the first patient is detected based on the plurality of monitoring parameters over the first observation window, wherein the first event is one of case start, maintenance start, emergence start, and case end;
based on the first event being detected, updating (617) the GUI to add the new phase to a first timeline of the plurality of timelines, the first timeline representing anesthesia delivery phases for the first patient;
identifying that a second event for the first patient is detected based on the plurality of monitoring parameters of the first patient over a second observation window;
determining, based on the first event and the second event, that the first event or the second event is a false positive event, and in response, updating the GUI to adjust the first timeline to reflect the false positive event.

12. The method of claim 11, wherein the first event is emergence start, the new phase is emergence, and the second event is maintenance start, wherein determining, based on the first event and the second event, that the first event or the second event is a false positive event comprises determining that the emergence start is the false positive event based on the maintenance start being detected after the emergence start was detected (830), and wherein updating the GUI to adjust the first timeline to reflect the false positive event comprises removing (834) emergence from the first timeline and extending a duration of maintenance on the first timeline.

13. The method of claim 11, wherein the first event is maintenance start, the new phase is maintenance, and the second event is case start, wherein determining, based on the first event and the second event, that the first event or the second event is a false positive event comprises determining that the maintenance start is the false positive event based on the case start being detected after the maintenance start was detected (836), and wherein updating the GUI to adjust the first timeline to reflect the false positive event comprises removing (838) maintenance from the first timeline and plotting induction on the first timeline.

14. The method of claim 11, wherein identifying that the first event is detected comprises:
applying (702) a first subset of rules to determine if case start is detected responsive to an end of a first standby event of the anesthesia delivery machine;
applying (704, 706) a second subset of rules and a third subset of rules in parallel to determine if maintenance start is detected;
applying (708, 710) a fourth subset of rules and a fifth subset of rules in parallel to determine if emergence start is detected; and
applying (712) a sixth subset of rules to determine if case end is detected responsive to a start of a second standby event of the anesthesia delivery machine.

15. The method of claim 11, wherein the GUI comprises:
an operating room status panel (1002) indicating a total number of patients in each phase of anesthesia delivery; and
wherein each timeline of the plurality of timelines (1010) is color-coded to indicate different phases of anesthesia delivery, the different phases comprising induction, maintenance, and emergence.
